# EUROPEAN PATENT SPECIFICATION

(11) **EP 1 128 790 B1**
(45) Date of publication and mention of the grant of the patent: **07.05.2003**
(21) Application number: 98961731.1
(22) Date of filing: 10.11.1998
(51) Int. Cl.: A61F 13/15

(54) **DISPOSABLE PULL-ON GARMENT HAVING IMPROVED DISPOSAL DEVICE**
WEGWERFERZIEHUNGSHÖSCHEN MIT BEFESTIGUNGSMITTEL ZUM WEGWERFEN
ARTICLE JETABLE FACILE A ENFILER ET DISPOSITIF D'ELIMINATION AMELIORE

(43) Date of publication of application: 05.09.2001
(73) Proprietor: The Procter & Gamble Company, Cincinnati, Ohio 45202 (US)
(72) Inventor: HAWKINS, Craig, Andrew, Ashiya-shi 659-0087 (JP)
(74) Representative: Hirsch, Uwe Thomas
(86) International application number: US9823883
(87) International publication number: WO00027328

(56) References cited:
- EP-A- 0 623 330
- EP-A- 0 861 642
- US-A- 5 626 573

## Description

### FIELD

The present invention relates to disposable pull-on garments. Examples of such disposable pull-on garments include disposable underwear, pull-on diapers, training pants, and disposable panties for menstrual use. The present invention more particularly relates to unitary disposable absorbent pull-on garments such as pull-on diapers, training pants, incontinent pull-on briefs, and the like, which provide improved disposal handling after having been soiled.

### BACKGROUND

Infants and other incontinent individuals wear disposable garments such as diapers to receive and contain urine and other body exudates. Disposable pull-on garments having fixed sides, which are also called "pant type" garments, have become popular for use on children able to walk and often who are toilet training. These pull-on garments have side panels with edges that are seamed together to form two leg openings and a waist opening. In order to contain body exudates as well as fit a wide variety of body shapes and sizes, these pull-on garments need to fit snugly about the waist and legs of the wearer without drooping, sagging or sliding down from its position on the torso. Examples of these pull-on garments are disclosed, for example, in U.S. Patent No. 5,171,239 issued to Igaue et al. on December 15; 1992, U.S. Patent No. 4,610,681 issued to Strohbeen et al. on September 9, 1986; U.S. Patent No. 4,940,464 issued to Van Gompel et al. on July 10, 1990; U.S. Patent No. 5,246,433 to issued Hasse et al. on September 21, 1993; U.S. Patent No. 5,569,234 issued to Buell et al. on October 29, 1996; and WO 96/31176 (Ashton) published on October 10, 1996.

After a pull-on garment has been soiled by urine or body exudes, it is removed from the wearer's body by tearing open the seams of the side panels. The removed pull-on garment then is folded so that the soiled portion is wrapped inside for disposal. To prevent the soiled pull-on garment from being unfolded and/or to keep the soiled portion inside, it is desired that such pull-on garment has a disposal means or device which secures or keeps the soiled garment in the folded state. Examples of such pull-on garments are disclosed in U.S. Patent No. 5,575,784 issued to Ames-Ooten et al. on November 19, 1996; European Patent Publication No. EP623330 (Hayase et al.) published on November 9, 1994; European Patent Publication No. EP0732094 (Toyoda) published on September 18, 1996; and Japanese Laid-open Patent Publication No. H8-117278 (Tabata) published on May 14, 1996.

EP-A-0 861 642 discloses a disposal securing means provided on a backsheet of body fluids absorbent garment for disposal thereof, which comprises a first strip section bonded to the backsheet, a second strip section bonded to the backsheet, and a third strip section having a proximal end portion connected to at least one of the first and second strip sections and a free end portion wherein the third strip section includes a stretchable extend defined between these opposite end portions.

However, such conventional pull-on garments tend to cause leakage of contained urine or body exudes from the side edge portions of the garments especially when the soiled garments are not appropriately folded and/or secured. For example, if a soiled garment is secured loosely in a folded state, the side edge portions of the garment tend to stick out thereby causing leakage of the contents.

Thus, none of the existing art provides all of the advantages and benefits of the present invention.

### SUMMARY

The present invention is directed to a disposable pull-on garment having a front region, a back region and a crotch region between the front region and the back region. The disposable pull-on garment includes a chassis provided in the front, back and crotch regions. The chassis has edge lines in the front and back regions and includes a liquid pervious topsheet, a liquid impervious backsheet associated with the topsheet, and an absorbent core disposed between the topsheet and the backsheet. The disposable pull-on garment further includes at least one pair of ear panels extending laterally outward from the chassis in the front or back region. The ear panels are joined to the chassis to form two leg openings and a waist opening. The disposable pull-on garment further includes a disposal device joined to the backsheet in the crotch region. The disposal device includes a first securing means which is capable of securing the disposable pull-on garment in a convenient disposal configuration after the garment has been soiled. The convenient disposal configuration is formed by securing a part of the ear panels to the backsheet through the first securing means. The disposal device has a means for protecting the first securing means prior to an intended use of the first securing means.

These and other features, aspects, and advantages of the present invention will become evident to those skilled in the art from reading of the present disclosure.

### BRIEF DESCRIPTION OF THE DRAWINGS

While the specification concludes with claims particularly pointing out and distinctly claiming the invention, it is believed that the invention will be better understood from the following description of preferred embodiments which is taken in conjunction with the accompanying drawings and which like designations are used to designate substantially identical elements, and in which:
Fig. 1 is a perspective view of one preferred embodiment of the disposable pull-on garment of the present invention in a typical in use configuration;
Fig. 2 is a perspective view of another preferred embodiment of the disposable pull-on garment of the present invention in a typical in use configuration;
Fig. 3 is a simplified plan view of the embodiment shown in Fig. 2 in its flat uncontracted condition showing the body-facing side the garment;
Fig. 4 is a cross-sectional view of a preferred embodiment taken along the section line 4-4 of Fig. 3;
Fig. 5 is a cross-sectional view of an elastic member 70 of a preferred embodiment;
Fig. 6 is a simplified plan view of the embodiment shown in Fig. 2 in its flat uncontracted condition showing the outer-facing side of the garment;
Fig. 7 is a cross-sectional view of one preferred disposal device;
Fig. 8 is a cross-sectional view showing a part of another preferred disposal device;
Fig. 9 is a cross-sectional view showing a part of yet another preferred disposal device;
Fig. 10 is a cross-sectional view showing a part of still another preferred disposal device;
Fig. 11 is a cross-sectional view of yet another preferred disposal device;
Fig. 12 is a cross-sectional view of still another preferred disposal device;
Fig. 13 is a perspective view of the pull-on garment shown in Fig. 2 when it is folded after having been soiled;
Fig. 14 is a perspective view of the pull-on garment shown in Fig. 2 when it is secured in a convenient disposal configuration;
Fig. 15 is a cross-sectional view of yet another preferred disposal device;
Fig. 16 is a cross-sectional view of the unfolded structure of the disposal device shown in Fig. 15;
Fig. 17 is a cross-sectional view of still another preferred disposal device; and
Fig. 18 is a cross-sectional view of yet another preferred disposal device.

### DETAILED DESCRIPTION

Citation of any reference is not an admission regarding any determination as to its availability as prior art to the claimed invention.

Herein, "comprise" means that other element(s) and step(s) which do not affect the end result can be added. These terms encompass the terms "consisting of" and "consisting essentially of".

Herein, "pull-on garment" refers to articles of wear which have a defined waist opening and a pair of leg openings and which are pulled onto the body of the wearer by inserting the legs into the leg openings and pulling the article up over the waist.

Herein, "disposable" describes garments which are not intended to be laundered or otherwise restored or reused as a garment (i.e., they are intended to be discarded after a single use and, preferably, to be recycled, composted or otherwise disposed of in an environmentally compatible manner).

Herein, "pull-on diaper" refers to pull-on garments generally worn by infants and other incontinent individuals to absorb and contain urine and feces. It should be understood, however, that the present invention is also applicable to other pull-on garments such as training pants, incontinent briefs, feminine hygiene garments or panties, and the like.

Herein, "front region" refers to one of the laterally divided regions of pull-on garments which does not form any part of the leg opening. When the pull-on garment is put on a wearer, the front region is positioned at the front of the wearer.

Herein, "back region" refers to one of the laterally divided regions of pull-on garments which does not form any part of the leg opening. When the pull-on garment is put on a wearer, the back region is positioned at the back of the wearer.

Herein, "crotch region" refers to the region which interposed between the front region and the back region of pull-on garments.

Herein, "panel" denotes an area or element of the pull-on garment. (While a panel is typically a distinct area or element, a panel may coincide (functionally correspond) somewhat with an adjacent panel.)

Herein, "joined" or "joining" encompasses configurations whereby an element is directly secured to another by affixing the element directly to the other element, and configurations whereby the element is indirectly secured to the other element by affixing the element to intermediate member(s) which in turn are affixed to the other element.

Herein, "engaging elements" refer to the elements of a hook fastening material which are intended to mechanically engage the fibrous elements of a complementary surface, such as loop fastening materials. The engaging elements may also be referred to as "male" elements.

Herein, "hook" should be non-limiting in the sense that the engaging elements may comprise any shapes as are know in the art so long as they are adapted to engage a complementary surface, such as a loop fastening material or other fibrous material. Examples of useful shapes include, but are not limited to, T-hooks, J-hooks, mushroom, and the like.

Herein, "loop fastening material" means a material having a plurality of fiber elements that are capable of engaging the engaging elements. Such materials are well known to one skilled in the art and include fibrous material, woven and nonwoven. Suitable loop fastening material may be manufactured from a wide range of materials to provide fiber elements, preferably loops. Such suitable materials include nylon, polyester, polypropylene, or any combination of these materials. Suitable examples include, e.g., the commercially available material designated "Scotchmate" brand nylon woven loop No. SJ3401 available from Minnesota Mining and Manufacturing Company, St. Pual, Minnesota, U.S.A.

Herein, "uncontracted state" is used to describe states of pull-on garments in its unseamed (i.e., seams are removed), flat and relaxed condition wherein all elastic materials used are removed therefrom.

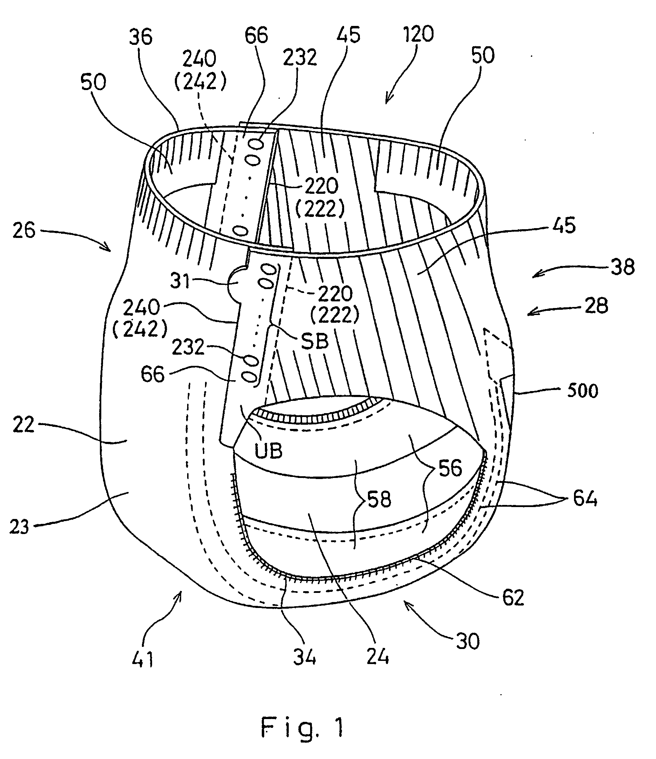

Fig. 1 shows one preferred embodiment of a disposable pull-on garment of the present invention (e.g., a unitary disposable pull-on diaper 120). Referring to Fig. 1, the disposable pull-on garment 120 of the present invention has a front region 26; a back region 28 and a crotch region 30 between the front region 26 and the back region 28. A chassis 41 is provided in the front, back and crotch regions 26, 28 and 30. The chassis 41 includes a liquid pervious topsheet 24, a liquid impervious backsheet 22 associated with the topsheet 24, and an absorbent core 25 (not shown in Fig. 1) disposed between the topsheet 24 and the backsheet 22. The chassis 41 has side edges 220 which form edge lines 222 in the front region 26.

The pull-on garment 120 of the invention further includes at least one pair of ear panels 45 each extending laterally outward from the corresponding sides of the chassis 41. Preferably, the ear panels 45 are extensible at least in the lateral direction. Each of the ear panels 45 has an outermost edge 240 which forms an outermost edge line 242. At least one of the outermost edge lines 242 has a nonuniform lateral distance from the longitudinal center line 100 (not shown in Fig. 1) in the uncontracted state of the garment 120.

In a preferred embodiment, the ear panels 45 continuously extend from the corresponding sides of the chassis 41 in the back region 28 to the corresponding side edges 220 of the chassis 41 in the front region 26 as shown in Fig. 1. Alternatively, the ear panels 45 may continuously extend from the corresponding sides of the chassis 41 in the front region 26 to the corresponding side edges of the chassis 41 in the back region 28 (not shown in Fig. 1).

The pull-on garment 120 of the invention has the ear panels 45 joined to the chassis 41 to form two leg openings 34 and a waist opening 36. Preferably, the pull-on garment 120 further includes seams 232 each joining the chassis 41 and the ear panels 45 along the corresponding edge lines 222 and 242 to form the two leg openings 34 and the waist opening 36.

In a preferred embodiment, at least one of the ear panels 45 having, along the seam 232, a substantially bonded portion SB starting from the waist opening 36 and an unbonded portion UB starting from the leg opening 34. Preferably, the ratio in length of the unbonded portion to the substantially bonded portion is between about 4:96 and 20:80.

The disposable pull-on garment 120 of the present invention further includes a disposal device 500 joined to the outer-facing surface 23 of the backsheet 22 in the crotch region 30. After the disposable pull-on garment 120 has been soiled, the disposal device 500 is used for securing each part of the ear panels 45 to the backsheet 22 so that the disposable pull-on garment 120 can be secured in a configuration that provides a convenient disposal. Preferred fastener devices which are applicable as the disposal device 500 will be described in detail hereinafter.

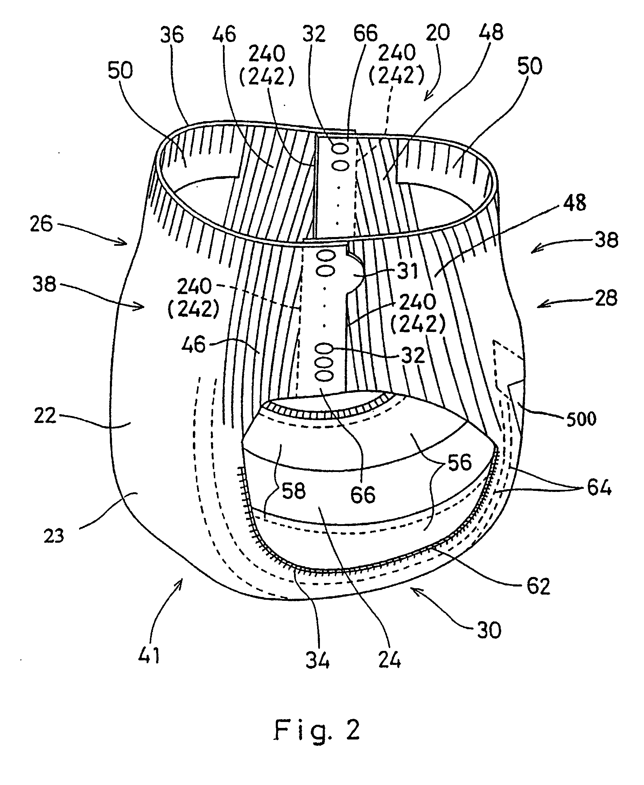

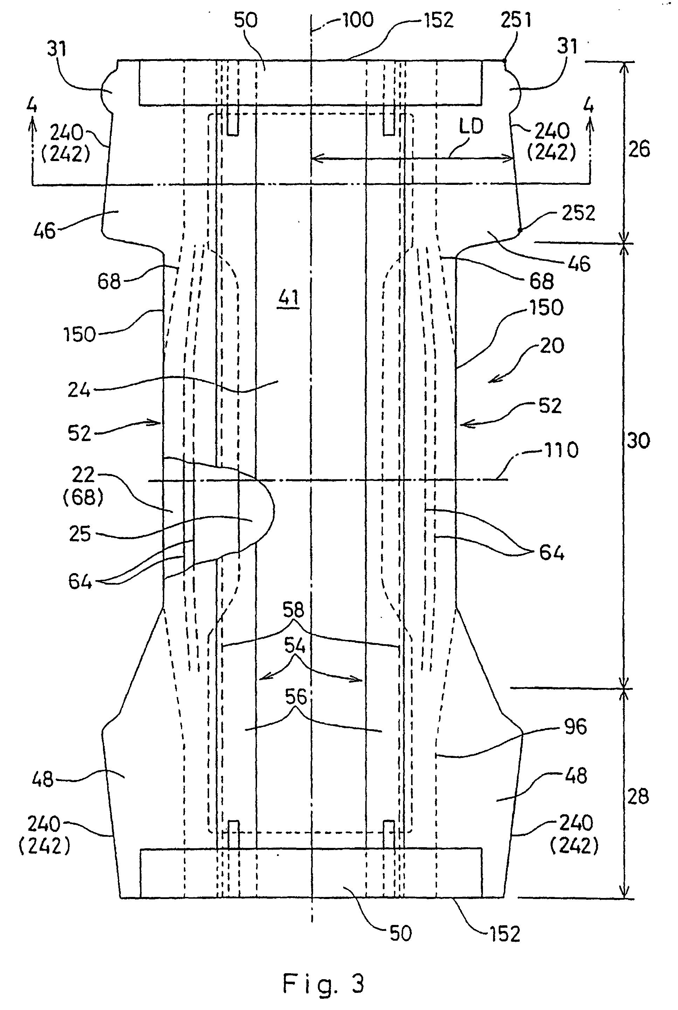

Fig. 2 shows another preferred embodiment of a disposable pull-on garment of the present invention (e.g., a unitary disposable pull-on diaper 20). Referring to Fig. 2, the disposable pull-on garment 20 includes a pair of front ear panels 46 each extending laterally outward from the corresponding sides of the chassis 41 in the front region 26, and a pair of extensible back ear panels 48 each extending laterally outward from the corresponding sides of the chassis 41 in the back region 28. Preferably, the ear panels 46 and 48 are extensible at least in the lateral direction. Each of the ear panels 46 and 48 has an outermost edge 240 which forms an outermost edge line 242. At least one of the outermost edge lines 242 has a nonuniform lateral distance LD from the longitudinal center line 100 (not shown in Fig. 2 but in Fig. 3) in the uncontracted state of the garment 20. The pull-on garment 20 further includes seams 32 each joining the front and back ear panels 46 and 48 along the corresponding edge lines 242 to form the two leg openings 34 and the waist opening 36.

The disposable pull-on garment 20 further includes the disposal device 500 joined to the outer-facing surface 23 of the backsheet 22 in the crotch region 30. After the disposable pull-on garment 20 has been soiled, the disposal device 500 is used for securing one pair of the ear panels 46 or 48 to the backsheet 22 so that the disposable pull-on garment 20 can be secured in a configuration that provides a convenient disposal. Preferred fastener devices which are applicable as the disposal device 500 will be described in detail hereinafter.

In a preferred embodiment, at least one of, more preferably both of, the pairs of the ear panels 45, 46 and 48 are elastically extensible in at least the lateral direction. In alternative embodiments, the ear panels 45, 46 and 48 are elastically extensible both in the lateral and longitudinal directions. Herein, "extensible" refers to materials that are capable of extending in at least one direction to a certain degree without undue rupture. Herein, "elasticity" and "elastically extensible" refer to extensible materials that have the ability to return to approximately their original dimensions after the force that extended the material is removed. Herein, any material or element described as "extensible" may also be elastically extensible unless otherwise provided. The extensible ear panels 45, 46 and 48 provide a more comfortable and contouring fit by initially conformably fitting the pull-on garment to the wearer and sustaining this fit throughout the time of wear well past when the pull-on garment has been loaded with exudates since the ear panels 45, 46 and/or 48 allow the sides of the pull-on garment to expand and contract.

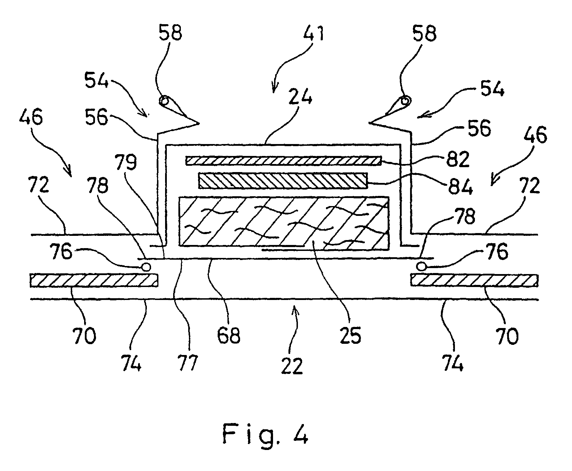

The ear panels 45, 46 and 48 may be formed by unitary elements of the pull-on garment 20 or 120 (i.e., they are not separately manipulative elements secured to the pull-on garment 20 or 120, but rather are formed from and are extensions of one or more of the various layers of the pull-on garment). In a preferred embodiment, each of the ear panels 45, 46 and 48 is a projected member of the chassis 41 (more clearly shown in Fig. 3). Preferably, the ear panels 45, 46 and 48 include at least one unitary element or a continuous sheet material (e.g. the nonwoven outer cover 74 in Fig. 4) that forms a part of the chassis 41 and continuously extends into the ear panels 45, 46 and 48. Alternatively, the ear panels 45, 46 and 48 may be discrete members (not shown in Figs.) which do not have any unitary element that forms a part of the chassis 41, and may be formed by joining the discrete members to the corresponding sides of the chassis 41.

In a preferred embodiment, the pull-on garment 20 or 120 further includes seam panels 66 each extending laterally outward from each of the ear panels 45, 46 and 48; and tear open tabs 31 each extending laterally outward from the seam panel 66. In a preferred embodiment, each of the seam panels 66 is an extension of the corresponding ear panels 45, 46 and 48, or at least one of the component elements used therein, or any other combination of the elements. More preferably, each of the tear open tabs 31 is also an extension of the corresponding seam panel 66 or at least one of its component elements used therein, or any other combination of its elements.

In a preferred embodiment, the corresponding edge portions of the chassis 41 and/or the ear panels 45, 46 and 48 are seamed directly or indirectly (e.g., through the seam panels 66), in an overlaping manner to make an overlapped seam structure. Alternatively. the front and ear panels 46 and 48 can be seamed in a butt seam manner (not shown in Figs.). The bonding of the seams 32 can be performed by any suitable means known in the art appropriate for the specific materials employed in the chassis 41 and/or the ear panels 45, 46 and 48. Thus, sonic sealing, heat sealing, pressure bonding, adhesive or cohesive bonding, sewing, autogeneous bonding, and the like may be appropriate techniques. Preferably, the seam panels 66 are joined by a predetermined pattern of heat/pressure or ultrasonic welds which withstands the forces and stresses generated on the garment 20 or 120 during wear.

A continuous belt 38 is formed by the ear panels 45, 46 and 48, and a part of the chassis 41 about the waist opening 36 as shown in Figs. 1 and 2. Preferably, elasticized waist bands 50 are provided in both the front region 26 and the back region 28. The continuous belt 38 acts to dynamically create fitment forces in the pull-on garment 20 or 120 when positioned on the wearer, to maintain the pull-on garment 20 or 120 on the wearer even when loaded with body exudates thus keeping the absorbent core 25 (not shown in Fig. 2) in close proximity to the wearer, and to distribute the forces dynamically generated during wear about the waist thereby providing supplemental support for the absorbent core 25 without binding or bunching the absorbent core 25.

Fig. 3 is a partially cut-away plan view of the pull-on garment 20 of Fig. 2 in its uncontracted state (except in the ear panels 46 and 48 which are left in their relaxed condition) with the topsheet 24 facing the viewer, prior to the ear panels 46 and 48 being joined together by the seams 32. The pull-on garment 20 has the front region 26, the back region 28 opposed to the front region 26, the crotch region 30 positioned between the front region 26 and the back region 28, and a periphery which is defined by the outer perimeter or edges of the pull-on garment 20 in which the side edges are designated 115 and 240, and the end edges or waist edges are designated 152. The topsheet 24 has the body-facing surface of the pull-on garment 20 which is positioned adjacent to the wearer's body during use. The backsheet 22 has the outer-facing surface of the pull-on garment 20 which is positioned away from the wearer's body. The pull-on garment 20 includes the chassis 41 including the liquid pervious topsheet 24, the liquid impervious backsheet 22 associated with the topsheet 24, and the absorbent core 25 positioned between the topsheet 24 and the backsheet 22. The garment 20 further includes the front and back ear panels 46 and 48 extending laterally outward from the chassis 41, the elasticized leg cuffs 52, and the elasticized waistbands 50. The topsheet 24 and the backsheet 22 have length and width dimensions generally larger than those of the absorbent core 25. The topsheet 24 and the backsheet 22 extend beyond the edges of the absorbent core 25 to thereby form the side edges 115 and the waist edges 152 of the garment 20. The liquid impervious backsheet 22 preferably includes a liquid impervious plastic film 68.

The pull-on garment 20 also has two centerlines, a longitudinal centerline 100 and a transverse centerline 110. Herein, "longitudinal" refers to a line, axis, or direction in the plane of the pull-on garment 20 that is generally aligned with (e.g. approximately parallel with) a vertical plane which bisects a standing wearer into left and right halves when the pull-on garment 20 is worn. Herein, "transverse" and "lateral" are interchangeable and refer to a line, axis or direction which lies within the plane of the pull-on garment that is generally perpendicular to the longitudinal direction (which divides the wearer into front and back body halves). The pull-on garment 20 and component materials thereof also have a body-facing surface which faces the skin of wearer in use and an outer-facing surface which is the opposite surface to the body-facing surface.

While the topsheet 24, the backsheet 22, and the absorbent core 25 may be assembled in a variety of well known configurations, exemplary chassis configurations are described generally in U.S. Patent 3,860,003 entitled "Contractible Side Portions for Disposable Diaper" which issued to Kenneth B. Buell on January 14, 1975; and U.S. Patent 5,151,092 entitled "Absorbent Article With Dynamic Elastic Waist Feature Having A Predisposed Resilient Flexural Hinge" which issued to Kenneth B. Buell et al., on September 29, 1992.

Fig. 4 is a cross-sectional view of a preferred embodiment taken along the section line 4-4 of Fig. 3. The pull-on garment 20 includes the chassis 41 including the liquid pervious topsheet 24, the liquid impervious backsheet 22 associated with the topsheet 24, and the absorbent core 25 positioned between the topsheet 24 and the backsheet 22. The pull-on garment further includes the front ear panels 46 each extending laterally outward from the chassis 41, and an inner barrier cuffs 54. Although Fig. 4 depicts only the structure of the front ear panel 46 and the chassis 41 in the front region 26, preferably a similar structure is also provided in the back region 28. In a preferred embodiment, each of the front ear panels 46 is formed by a lamination of an extended part 72 of the barrier flap 56, an elastic member 70 and the nonwoven outer cover 74. The elastic member 70 includes a plane elastomeric material 124 (not shown in Fig. 4). Herein, "plane elastomeric material" refers to elastomeric materials which continuously extend in two dimensional directions. Preferred plane elastomeric materials include a scrim, a perforated (or apertures formed) film, an elastomeric woven or nonwoven, and the like. In a preferred embodiment, the plane elastomeric material 124 includes at least a portion that has a nonuniform lateral width.

The absorbent core 25 can be any absorbent member which is generally compressible, conformable, non-irritating to the wearer's skin, and capable of absorbing and retaining liquids such as urine and other certain body exudates. The absorbent core 25 may be manufactured in a wide variety of sizes and shapes (e.g., rectangular, hourglass, "T''-shaped, asymmetric, etc.) and from a wide variety of liquid-absorbent materials commonly used in disposable pull-on garments and other absorbent articles such as comminuted wood pulp which is generally referred to as airfelt. Examples of other suitable absorbent materials include creped cellulose wadding; meltblown polymers including coform; chemically stiffened, modified or cross-linked cellulosic fibers; tissue including tissue wraps and tissue laminates; absorbent foams; absorbent sponges; superabsorbent polymers; absorbent gelling materials; or any equivalent material or combinations of materials.

The configuration and construction of the absorbent core 25 may vary (e.g., the absorbent core 25 may have varying caliper zones, a hydrophilic gradient, a superabsorbent gradient, or lower average density and lower average basis weight acquisition zones; or may include one or more layers or structures). Further, the size and absorbent capacity of the absorbent core 25 may also be varied to accommodate wearers ranging from infants through adults. However, the total absorbent capacity of the absorbent core 25 should be compatible with the design loading and the intended use of the garment 20.

A preferred embodiment of the garment 20 has an asymmetric, modified hourglass-shaped absorbent core 25 having ears in the front and back waist regions 26 and 28. Other exemplary absorbent structures for use as the absorbent core 25 that have achieved wide acceptance and commercial success are described in U.S. Patent No. 4,610,678 entitled "High-Density Absorbent Structures" issued to Weisman et al. on September 9, 1986; U.S. Patent No. 4,673,402 entitled "Absorbent Articles With Dual-Layered Cores" issued to Weisman et al. on June 16, 1987; U.S. Patent No. 4,888,231 entitled "Absorbent Core Having A Dusting Layer" issued to Angstadt on December 19, 1989; and U.S. Patent No. 4,834,735, entitled "High Density Absorbent Members Having Lower Density and Lower Basis Weight Acquisition Zones", issued to Alemany et al. on May 30, 1989.

The chassis 41 may further include an acquisition/distribution core 84 of chemically stiffened fibers positioned over the absorbent core 25, thereby forming a dual core system. In a preferred embodiment, the fibers are hydrophilic chemically stiffened cellulosic fibers. Herein, "chemically stiffened fibers" means any fibers which have been stiffened by chemical means to increase stiffness of the fibers under both dry and aqueous conditions. Such means include the addition of chemical stiffening agents which, for example, coat and/or impregnate the fibers. Such means also include the stiffening of the fibers by altering the chemical structure of the fibers themselves, e.g., by cross-linking polymer chains.

The fibers utilized in the acquisition/distribution core 84 can also be stiffened by means of chemical reaction. For example, crosslinking agents can be applied to the fibers which, subsequent to application, are caused to chemically form intrafiber crosslink bonds. These crosslink bonds can increase stiffness of the fibers. Whereas the utilization of intrafiber crosslink bonds to chemically stiffen the fibers is preferred, it is not meant to exclude other types of reactions for chemical stiffening of the fibers.

In the more preferred stiffened fibers, chemical processing includes intrafiber crosslinking with crosslinking agents while such fibers are in a relatively dehydrated, defibrated (i.e. individualized), twisted, curled condition. Suitable chemical stiffening agents include monomeric crosslinking agents including, but not limited to, C₂-C₈ dialdehydes and C₂-C₈ monoaldehydes having an acid functionality can be employed to form the cosslinking solution. These compounds are capable of reacting with at least two hydroxyl groups in a single cellulose chain or on proximately located cellulose chains in a single fiber. Such crosslinking agents contemplated for use in preparing the stiffened cellulose fibers include, but are not limited to, glutaraldehyde, glyoxal, formaldehyde, and glyoxylic acid. Other suitable stiffening agents are polycarboxylates, such as citric acid. The polycarboxylic stiffening agents and a process for making stiffened fibers from them are described in U.S. Patent No. 5,190,563, entitled "Process for Preparing Individualized, Polycarboxylic Acid crosslinked Fibers" issued to Herron, on March 2, 1993. The effect of crosslinking under these conditions is to form fibers which are stiffened and which tend to retain their twisted, curled configuration during use in the absorbent articles herein. Such fibers, and processes for making them are described in the above cited patents.

Preferred dual core systems are disclosed in U.S. Patent No. 5,234,423, entitled "Absorbent Article With Elastic Waist Feature and Enhanced Absorbency" issued to Alemany et al., on August 10, 1993; and in U.S. Patent No. 5,147,345, entitled "High Efficiency Absorbent Articles For Incontinence Management" issued to Young, LaVon and Taylor on September 15, 1992. In a preferred embodiment, the acquisition/distribution core 84 includes chemically treated stiffened cellulosic fiber material, available from Weyerhaeuser Co. (U.S.A.) under the trade designation of "CMC". Preferably, the acquisition/distribution core 84 has a basis weight of from about 40 g/m² to about 400 g/m², more preferably from about 75 g/m² to about 300 g/m².

More preferably, the chassis 22 further includes an acquisition/distribution layer 82 between the topsheet 24 and the acquisition/distribution core 84 as shown in Fig. 4. The acquisition/distribution layer 82 is provided to help reduce the tendency for surface wetness of the topsheet 24. The acquisition/distribution layer 82 preferably includes carded, resin bonded hiloft nonwoven materials such as, for example, available as Code No. FT-6860 from Polymer Group, Inc., North America (Landisiville, New Jersey, U.S.A.), which is made of polyethylene telephthalate fibers of 6 dtex, and has a basis weight of about 43 g/m². A preferable example for the acquisition/distribution layer 82 and the acquisition/distribution core 84 is disclosed in EP 0797968A1 (Kurt et al.) published on October 1, 1997.

The topsheet 24 is preferably compliant, soft feeling, and non-irritating to the wearer's skin. Further, the topsheet 24 is liquid pervious permitting liquids (e.g., urine) to readily penetrate through its thickness. A suitable topsheet 24 may be manufactured from a wide range of materials such as woven and nonwoven materials; polymeric materials such as apertured formed thermoplastic films, apertured plastic films, and hydroformed thermoplastic films; porous foams; reticulated foams; reticulated thermoplastic films; and thermoplastic scrims. Suitable woven and nonwoven materials can be included of natural fibers (e.g., wood or cotton fibers), synthetic fibers (e.g., polymeric fibers such as polyester, polypropylene, or polyethylene fibers) or from a combination of natural and synthetic fibers. The topsheet 24 is preferably made of a hydrophobic material to isolate the wearer's skin from liquids which have passed through the topsheet 24 and are contained in the absorbent core 25 (i.e., to prevent rewet). If the topsheet 24 is made of a hydrophobic material, at least the upper surface of the topsheet 24 is treated to be hydrophilic so that liquids will transfer through the topsheet more rapidly. This diminishes the likelihood that body exudates will flow off the topsheet 24 rather than being drawn through the topsheet 24 and being absorbed by the absorbent core 25. The topsheet 24 can be rendered hydrophilic by treating it with a surfactant. Suitable methods for treating the topsheet 24 with a surfactant include spraying the topsheet 24 material with the surfactant and immersing the material into the surfactant. A more detailed discussion of such a treatment and hydrophilicity is contained in U.S. Patent No. 4,988,344 entitled "Absorbent Articles with Multiple Layer Absorbent Layers" issued to Reising, et al. on January 29, 1991 and U.S. Patent No. 4,988,345 entitled "Absorbent Articles with Rapid Acquiring Absorbent Cores" issued to Reising on January 29, 1991.

In a preferred embodiment, the topsheet 24 is a nonwoven web that can provide reduced tendency for surface wetness; and consequently facilitate maintaining urine absorbed by the core 25 away from the user's skin, after wetting. One of the preferred topsheet materials is a thermobonded carded web which is available as Code No. P-8 from Fiberweb North America, Inc. (Simpsonville, South Carolina, U.S.A.). Another preferred topsheet material is available as Code No. S-2355 from Havix Co., Japan. This material is a bi-layer composite material, and made of two kinds of synthetic surfactant treated bicomponent fibers by using carding and air-through technologies. Yet another preferred topsheet material is a thermobonded carded web which is available as Code No. Profleece Style 040018007 from Amoco Fabrics, Inc. (Gronau, Germany).

Another preferred topsheet 24 includes an apertured formed film. Apertured formed films are preferred for the topsheet 24 because they are pervious to body exudates and yet non-absorbent and have a reduced tendency to allow liquids to pass back through and rewet the wearer's skin. Thus, the surface of the formed film which is in contact with the body remains dry, thereby reducing body soiling and creating a more comfortable feel for the wearer. Suitable formed films are described in U.S. Patent No. 3,929,135, entitled "Absorptive Structures Having Tapered Capillaries", issued to Thompson on December 30, 1975; U.S. Patent No. 4,324,246 entitled "Disposable Absorbent Article Having A Stain Resistant Topsheet", issued to Mullane, et al. on April 13, 1982; U.S. Patent No. 4,342,314 entitled "Resilient Plastic Web Exhibiting Fiber-Like Properties", issued to Radel. et al. on August 3, 1982; U.S. Patent No. 4,463,045 entitled "Macroscopically Expanded Three-Dimensional Plastic Web Exhibiting Non-Glossy Visible Surface and Cloth-Like Tactile Impression", issued to Ahr et al. on July 31, 1984; and U.S. 5,006,394 "Multilayer Polymeric Film" issued to Baird on April 9, 1991.

In a preferred embodiment, the backsheet 22 includes the liquid impervious film 68 as shown in, for example, Fig. 4. Preferably, the liquid impervious film 68 longitudinally extends in the front, back and crotch regions 26, 28 and 30. More preferably, the liquid impervious film 68 does not laterally extend into the at least one of the ear panels 46 or 48. The liquid impervious film 68 has a body-facing surface 79 and an outer-facing surface 77. The liquid impervious film 68 is impervious to liquids (e.g., urine) and is preferably manufactured from a thin plastic film. However, more preferably the plastic film permits vapors to escape from the garment 20. In a preferred embodiment, a microporous polyethylene film is used for the liquid impervious film 68. A suitable microporous polyethylene film is manufactured by Mitsui Toatsu Chemicals, Inc., Nagoya, Japan and marketed in the trade as PG-P. In a preferred embodiment, a disposable tape (not shown in Figs.) is additionally joined to the outer surface of the backsheet 22 to provide a convenient disposal after soiling.

A suitable material for the liquid impervious film 68 is a thermoplastic film having a thickness of from about 0.012 mm (0.5 mil) to about 0.051 mm (2.0 mils), preferably including polyethylene or polypropylene. Preferably, the liquid impervious film has a basis weight of from about 5 g/m² to about 35 g/m². However, it should be noted that other flexible liquid impervious materials may be used. Herein, "flexible" refers to materials which are compliant and which will readily conform to the general shape and contours of the wearer's body.

Preferably, the backsheet 22 further includes the nonwoven outer cover 74 which is joined with the outer-facing surface of the liquid impervious film 68 to form a laminate (i.e., the backsheet 22). The nonwoven outer cover 74 is positioned at the outermost portion of the garment 20 and covers at least a portion of the outermost portion of the garment 20. In a preferred embodiment, the nonwoven outer cover 74 covers almost all of the area of the outermost portion of the garment 20. The nonwoven outer cover 74 may be joined to the liquid impervious film 68 by any suitable attachment means known in the art. For example, the nonwoven outer cover 74 may be secured to the liquid impervious film 68 by a uniform continuous layer of adhesive, a patterned layer of adhesive, or an array of separate lines, spirals, or spots of adhesive. Suitable adhesives include a hotmelt adhesive obtainable from Nitta Findley Co., Ltd., Osaka, Japan as H-2128, and a hotmelt adhesive obtainable from H.B. Fuller Japan Co., Ltd., Osaka, Japan as JM-6064.

In a preferred embodiment, the nonwoven outer cover 74 is a carded nonwoven web, for example, obtainable from Havix Co., LTD., Gifu, Japan as E-2341. The nonwoven outer cover 74 is made of bi-component fibers of a polyethylene and a polypropylene. The ratio of PE/PP is about 50/50. The PE/PP bi-component fiber has the dimension of 2d x 51 mm. Another preferred carded nonwoven web is obtainable from Chisso Corp., Moriyama, Japan. The nonwoven outer cover 74 is also made of bi-component fibers of a polyethylene and a polypropylene. The ratio of PE/PP is about 50/50.

In another preferred embodiment, the nonwoven web is a spunbonded nonwoven web, for example, obtainable from Mitsui Petrochemical Industries, Ltd., Tokyo, Japan. The nonwoven web is made of bi-component fibers of a polyethylene and a polypropylene. The ratio of PE/PP is about 80/20. The PE/PP bi-component fiber has the thickness is approximately 2.3d.

The backsheet 22 is preferably positioned adjacent the outer-facing surface of the absorbent core 25 and is preferably joined thereto by any suitable attachment means known in the art. For example, the backsheet 22 may be secured to the absorbent core 25 by a uniform continuous layer of adhesive, a patterned layer of adhesive, or an array of separate lines, spirals, or spots of adhesive. Adhesives which have been found to be satisfactory are manufactured by H. B. Fuller Company of St. Paul, Minnesota, U.S.A., and marketed as HL-1358J. An example of a suitable attachment means including an open pattern network of filaments of adhesive is disclosed in U.S. Patent No. 4,573,986 entitled "Disposable Waste-Containment Garment", which issued to Minetola et al. on March 4, 1986. Another suitable attachment means including several lines of adhesive filaments swirled into a spiral pattern is illustrated by the apparatus and methods shown in U.S. Patent No. 3,911,173 issued to Sprague, Jr. on October 7, 1975; U.S. Patent No. 4,785,996 issued to Ziecker, et al. on November 22, 1978; and U.S. Patent No. 4,842,666 issued to Werenicz on June 27, 1989. Alternatively, the attachment means may include heat bonds, pressure bonds, ultrasonic bonds, dynamic mechanical bonds, or any other suitable attachment means or combinations of these attachment means as are known in the art.

In an alternative embodiment, the absorbent core 25 is not joined to the backsheet 22, and/or the topsheet 24 in order to provide greater extensibility in the front region 26 and the back region 28.

The pull-on garment 20 preferably further includes elasticized leg cuffs 52 for providing improved containment of liquids and other body exudates. The elasticized leg cuffs 52 may include several different embodiments for reducing the leakage of body exudates in the leg regions. (The leg cuffs can be and are sometimes also referred to as leg bands, side flaps, barrier cuffs, elastic cuffs or gasketing cuffs.) U.S. Patent 3,860,003 entitled "Contractable Side Portions for Disposable Diaper" issued to Buell on January 14, 1975, describes a disposable diaper which provides a contractible leg opening having a side flap and one or more elastic members to provide an elasticized leg cuff. U.S. Patent 4,909,803 entitled "Disposable Absorbent Article Having Elasticized Flaps" issued to Aziz et al. on March 20, 1990, describes a disposable diaper having "stand-up" elasticized flaps (barrier cuffs) to improve the containment of the leg regions. U.S. Patent 4,695,278 entitled "Absorbent Article Having Dual Cuffs" issued to Lawson on September 22, 1987; and U.S. Patent 4,795,454 entitled "Absorbent Article Having Leakage-Resistant Dual Cuffs" issued to Dragoo on January 3, 1989, describe disposable diapers having dual cuffs including a gasketing cuff and a barrier cuff. U.S. Patent 4,704,115 entitled "Disposable Waist Containment Garment" issued to Buell on November 3, 1987, discloses a disposable diaper or incontinence garment having side-edge-leakage-guard gutters configured to contain free liquids within the garment.

While each elasticized leg cuff 52 may be configured so as to be similar to any of the leg bands, side flaps, barrier cuffs, or elastic cuffs described above, it is preferred that the elasticized leg cuff 52 includes an elastic gasketing cuff 62 with one or more elastic strands 64 as shown in Fig. 3, which is described in the above-referred U.S. Patent Nos. 4,695,278 and 4,795,454. It is also preferred that each elasticized leg cuff 52 further includes inner barrier cuffs 54 each including a barrier flap 56 and a spacing means 58 which are described in the above-referenced U.S. Patent No. 4,909,803.

The pull-on garment 20 preferably further includes an elasticized waistband 50 that provides improved fit and containment. The elasticized waistband 50 is that portion or zone of the pull-on garment 20 which is intended to elastically expand and contract to dynamically fit the wearer's waist. The elasticized waistband 50 preferably extends longitudinally outwardly from the waist edge of the pull-on garment 20 toward the waist edge of the absorbent core 25. Preferably, the pull-on garment 20 has two elasticized waistbands 50, one positioned in the back region 28 and one positioned in the front region 26, although other pull-on garment embodiments can be constructed with a single elasticized waistband. The elasticized waistband 50 may be constructed in a number of different configurations including those described in U.S. Patent 4,515,595 entitled "Disposable Diapers with Elastically Contractible Waistbands" issued to Kievit et al. on May 7, 1985 and the above referenced U.S. Patent 5,151,092 issued to Buell.

The waistbands 50 may include materials that have been "prestrained" or "mechanically prestrained" (i.e., subjected to some degree of localized pattern mechanical stretching to permanently elongate the material). The materials may be prestrained using deep embossing techniques as are known in the art. Alternatively, the materials may be prestrained by directing the material through an incremental mechanical stretching system as described in U.S. Patent No. 5,330,458 entitled "Absorbent Article With Elastic Feature Having A Portion Mechanically Prestrained" issued to Buell et al., on July 19, 1994. The materials are then allowed to return to their substantially untensioned condition, thus forming a zero strain stretch material that is extensible, at least up to the point of initial stretching. Examples of zero strain materials are disclosed in U.S. Patent No. 2,075,189 issued to Galligan on March 30, 1937; U.S. Patent No. 3,025,199 issued to Harwood on March 13, 1962; U.S. Patent Nos. 4,107,364 and 4,209,563 issued to Sisson on August 15, 1978 and June 24, 1980, respectively; U.S. Patent No. 4,834,741 issued to Sabee on May 30, 1989; and U.S. Patent No. 5,151,092 issued to Buell et al., on September 29, 1992.

At least one of the ear panels 45, 46 and 48 includes the elastic member 70 as shown in Fig. 4. The elastic member 70 of the front ear panels 46 includes the elastomeric material 124 (not shown in Fig. 4) which preferably extends laterally outward from the chassis 41 to provide good fitness by generating the optimal retention (or sustained) force at the waist and side areas of the wearer. Preferably, the elastomeric material 124 is extensible in at least one direction, preferably in the lateral direction to generate a retention (or sustained) force that is optimal to prevent the pull-on garment 20 from drooping, sagging, or sliding down from its position on the torso without causing the red marking on the skin of the wearer. In a preferred embodiment, each of the ear panels 45, 46 and 48 includes the elastomeric material 124.

The elastic member 70 is operatively joined to at least one of the nonwoven webs 72 and 74 in the ear panels 45, 46 and 48 to allow the elastic member 70 to be elastically extensible in at least the lateral direction. In a preferred embodiment, the elastic member 70 is operatively joined to the nonwoven webs 72 and 74 by securing them to at least one, preferably both of the nonwoven webs 72 and 74 while in a substantially untensioned (zero strain) condition.

The elastic member 70 can be operatively joined to the nonwoven webs 72 and 74, by using either an intermittent bonding configuration or a substantially continuous bonding configuration. Herein, "intermittently" bonded laminate web means a laminate web wherein the plies are initially bonded to one another at discrete spaced apart points or a laminate web wherein the plies are substantially unbonded to one another at discrete spaced apart areas. Conversely, a "substantially continuously" bonded laminate web means a laminate web wherein the plies are initially bonded substantially continuously to one another throughout the areas of interface. It is preferred that the stretch laminate be bonded over all or a significant portion of the stretch laminate so that the inelastic webs (i.e., the nonwoven webs 72 and 74) elongate or draw without causing rupture, and the layers of the stretch laminates are preferably bonded in a configuration that maintains all of the layers of the stretch laminate in relatively close adherence to one another after the incremental mechanical stretching operation. Consequently, the elastic panel members and the other plies of the stretch laminate are preferably substantially continuously bonded together using an adhesive. In a particularly preferred embodiment, the adhesive selected is applied with a control coat spray pattern at a basis weight of about 7.0 grams/square m. The adhesive pattern width is about 6.0 cm. The adhesive is preferably an adhesive such as is available from Nitta Findley Co., Ltd., Osaka, Japan, under the designation H2085F. Alternatively, the elastic panel member and any other components of the stretch laminates may be intermittently or continuously bonded to one another using heat bonding, pressure bonding, ultrasonic bonding, dynamic mechanical bonding, or any other method as is known in the art.

After the elastic member 70 is operatively joined to at least one of the nonwoven webs 72 and 74, at least a portion of the resultant composite stretch laminate is then subjected to mechanical stretching sufficient to permanently elongate the non-elastic components which are, for example, the nonwoven webs 72 and 74. The composite stretch laminate is then allowed to return to its substantially untensioned condition. At least one pair of, preferably both of the ear panels 45, 46 and 48 is thus formed into "zero strain" stretch laminates. (Alternatively, the elastic member 70 could be operatively joined in a tensioned condition and then subjected to mechanical stretching; although this is not as preferred as a "zero strain" stretch laminate.) Herein, "zero strain" stretch laminate refers to a laminate included of at least two plies of material which are secured to one another along at least a portion of their coextensive surfaces while in a substantially untensioned ("zero strain") condition; one of the plies including a material which is stretchable and elastomeric (i.e., will return substantially to its untensioned dimensions after an applied tensile force has been released) and a second ply which is elongatable (but not necessarily elastomeric) so that upon stretching the second ply will be, at least to a degree, permanently elongated so that upon release of the applied tensile forces, it will not fully return to its original undeformed configuration. The resulting stretch laminate is thereby rendered elastically extensible, at least up to the point of initial stretching, in the direction of initial stretching. Particularly preferred methods and apparatus used for making stretch laminates utilize meshing corrugated rolls to mechanically stretch the components. Particularly preferred apparatus and methods are disclosed in U.S. Patent No. 5,167,897 issued to Weber et al. on December 1, 1992; U.S. Patent No. 5,156,793 issued to Buell et al. on October 20, 1990; and U.S. Patent No. 5,143,679 issued to Weber et al. on September 1, 1992.

The elastic member 70 is preferably joined to, more preferably directly secured to the respective edges 78 of the liquid impervious film (i.e., the liquid impervious film 68) through an adhesive 76 as shown in Fig. 4. In a preferred embodiment, while liquid impervious film 68 longitudinally extends in the front, back and crotch regions 26, 28 and 30, it does not laterally extend into at least one of, preferably each of the extensible ear panels 45, 46 and 48. In a more preferred embodiment, the elastic member 70 is joined to the respective edges 78 of the liquid impervious film 68 at the outer-facing surface 77 as shown in Fig. 4. In an alternative embodiment, the elastic member 70 may be joined to the respective edges 78 of the liquid impervious film 68 at the body-facing surface 79 (not shown in Figs.). Preferably, the adhesive 76 is applied in a spiral glue pattern. In a preferred embodiment, the adhesive 76 is a flexible adhesive with an amorphous and crystallizing component. Such a preferred adhesive is made by Nitta Findley Co., Ltd., Osaka, Japan, under the designation H2085F. Alternatively, the elastic member 70 may be joined to the respective edges 78 of the liquid impervious film 68 by any other bonding means known in the art which include heat bonds, pressure bonds, ultrasonic bonds, dynamic mechanical bonds, or combinations of these attachment means.

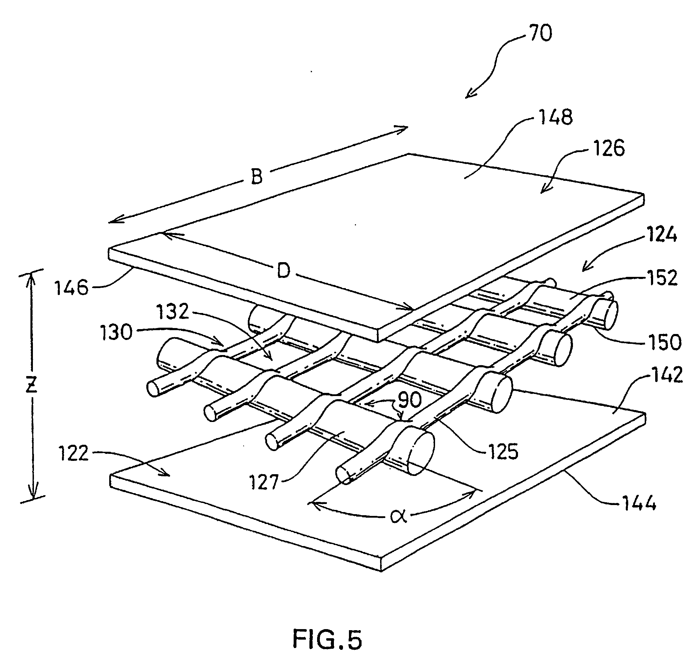

Referring to Fig. 5, the elastic member 70 includes the elastomeric material 124 having a first surface 150 and a second surface 152 opposing the first surface 150, and a first coverstock layer 122 which is joined to the first surface 150 of the elastomeric material 124. In a preferred embodiment, the first coverstock layer 122 is joined to the first surface 150 of the elastomeric material 124 by an adhesive (not shown in Fig. 5). More preferably, the elastic member 70 further includes a second coverstock layer 126 which is joined to the second surface 152 of the elastomeric material 124 by an adhesive (not shown in Fig. 5).

Preferably, the elastic member 70 is joined to the respective edges 78 of the liquid impervious film 68 at the outer-facing surface 77 as shown in Fig. 4. In an alternative embodiment, the elastic member 70 may be joined to the respective edges 78 of the liquid impervious film 68 at the body-facing surface 79 (not shown in Figs.).

The elastomeric material 124 may be formed in a wide variety of sizes, forms and shapes. In a preferred embodiment, the elastomeric material 124 is in the form of a continuous plane layer. Preferred forms of continuous plane layer include a scrim, a perforated (or apertures formed) film, an elastomeric woven or nonwoven, and the like. The continuous plane layer may take any shape which can be suitably provided in the ear panels. Preferred shapes of continuous plane layer include a quadrilateral including a rectangle and a square, a trapezoid, and the other polygons. In an alternative embodiment, the elastomeric material 124 is in the form of discrete strands (or strings) which are not connected each other.

Elastomeric materials which have been found to be especially suitable for the elastomeric material 124 are styrenic block copolymer based scrim materials, perforated (or apertured) elastic films, preferably with a thickness of from about 0.05 mm to about 1.0 mm (0.002 inch - 0.039 inch). Other suitable elastomeric materials for the elastomeric material 124 include "live" synthetic or natural rubber, other synthetic or natural rubber foams, elastomeric films (including heat shrinkable elastomeric films), elastomeric woven or nonwoven webs, elastomeric composites, or the like.

In the preferred embodiment shown in Fig. 5, the elastomeric scrim 124 has a plurality of first strands 125 and a plurality of second strands 127. The plurality of first strands 125 intersect the plurality of second strands 127 at nodes 130 at a predetermined angle α, forming a net-like open structure having a plurality of apertures 132. Each aperture 132 is defined by at least two adjacent first strands and at least two adjacent second strands, so that the apertures 132 are substantially rectangular in shape. Other configurations of the apertures 132, such as parallelograms, squares, or circular arc segments, can also be provided. Preferably, the first and second strands 125 and 127 are substantially straight and substantially parallel to one another. Preferably, the first strands 125 intersect the second strands 127 at nodes 130 such that the angle α is about 90 degrees. The first and second strands 125 and 127 are preferably joined or bonded at nodes 90.

A preferred elastomeric scrim 124 is manufactured by the Conwed Plastics Company (Minneapolis, Minn., U.S.A.) under the designation XO2514. This material has about 12 elastic strands per inch in the structural direction B (i.e., the first strands 125) and about 7 elastic strands per inch in the structural direction D (i.e., the second strands 127).

In a alternative preferred embodiment, the elastomeric material 124 is a porous, macroscopically-expanded, three-dimensional elastomeric web (not shown in Figs). The elastomeric web preferably comprises a formed film having at least two polymeric layers, with at least one of the layers being an elastomer layer and at least one of the other layers being a substantially less elastomeric skin layer. A preferred porous elastomeric material 124 is available from Tredegar Film Products under the designation X-25007.

In the embodiment shown in Fig. 5, the elastic member 70 includes first and second coverstock layers 122 and 126, and elastomeric material 124 disposed in the first and second coverstock layers 122 and 126. The first coverstock layer 122 has an inner surface 142 and an outer surface 144. The inner surface 142 of the first coverstock layer 122 is the surface that is positioned facing the elastomeric material 124. The second coverstock layer 126 also has an inner surface 146 and an outer surface 148. The inner surface 146 of the second coverstock layer 126 is the surface that is positioned facing the elastomeric material 124. The elastomeric material 124 also has two planar surfaces, first surface 150 and second surface 152, each of which is substantially parallel with the planes of the first and second coverstock layers 122 and 126. The first surface 150 is that planar surface of the elastomeric material 124 that is most closely adjacent with the inner surface 142 of first coverstock layer 122. The second surface 152 is that planar surface of elastomeric material 124 that is most closely adjacent to the inner surface 146 of the second coverstock layer 126.

Since the elastic member 70 will be subjected to mechanical stretching before and during use, the first and second coverstock layers 122 and 126 preferably have a relatively high elongation at breaking, and are more preferably stretchable or elongatable, yet more preferably drawable (but not necessarily elastomeric), without undue (and preferably without any), tearing or ripping. Further, the first and second coverstock layers 122 and 126 are preferably compliant, soft feeling, and non-irritating to the wearer's skin and give the article the feel and comfort of a cloth garment. Suitable materials for the first and second coverstock layers 122. and 126 can be manufactured from a wide range of materials such as plastic films, apertured plastic films, woven or nonwoven webs of natural fibers (e.g., wood or cotton fibers), synthetic fibers (e.g., polyolefins, polyamides, polyester, polyethylene, or polypropylene fibers), or a combination of natural and/or synthetic fibers, or coated woven or nonwoven webs.

Preferably, each of the first and second coverstock layers 122 and 126 is an identical consolidated nonwoven material. An exemplary preferred nonwoven material is manufactured by the Fiberweb North America, Inc. (Simpsonville, South Carolina, U.S.A.) under the designation Sofspan 200. This material has a basis weight of 25 g/m² before consolidation and a basis weight of about 63g/m2 after consolidation. Herein, "basis weight" is the weight of one square meter of planar web material. Alternatively, highly strainable nonwoven materials may be used. Alternatively, the first and second coverstock layers 122 and 126 need not be of identical materials, as long as the desired performance requirements, such as elastic performance, softness, flexibility, breathability and durability, are met. Herein, "consolidated nonwoven material" means a nonwoven material that has been gathered or necked under mechanical tension in the structural direction D so that the material can elongate in the structural direction D under low force.

The elastomeric material 124 and the first and second coverstock layers 122 and 126 are joined together, preferably by using an adhesive, to form the elastic member 70. A preferred method for making the elastic member 70 is described in the patent application WO 98/55298 entitled "Methods for Forming Extensible Laminate Structures" filed on June 6, 1997.

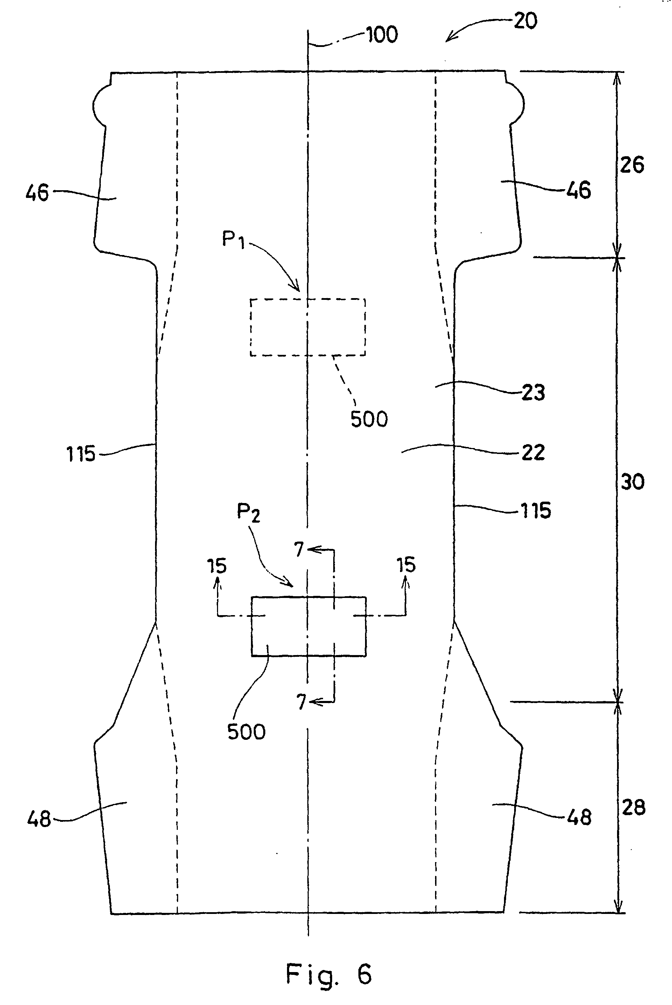

Fig. 6 is a simplified plan view of the embodiment shown in Fig. 2 in its flat uncontracted condition showing the outer-facing side 23 of the disposable pull-on garment 20. As shown in Fig. 6, the disposal device 500 is joined to the outer-facing surface 23 of the backsheet 22 in the crotch region 30.

The position of the disposal device 500 can be selected at any position within the crotch region 30 as long as the disposal device 500 can provide a convenient disposal by joining respective appropriate parts of the ear panels 46 or 48 to the backsheet 22. In one embodiment, the position of the disposal device 500 is selected at the position P1 which is on the longitudinal center line 100 and is close to the front region 26 as shown in Fig. 6. Alternatively and more preferably, the position of the disposal device 500 is selected at the position P2 which is on the longitudinal center line 100 and is close to the back region 28 as shown in Fig. 6. In Fig. 6, the side edges of the disposable pull-on garment 20 are designated by 115.

When the disposal device 500 is joined to the outer-facing surface 23 of the backsheet 22, the direction of the disposal device 500 can be selected at any direction as long as such direction does not disturb the convenient disposal function of the disposal device 500. Preferred directions are depicted in Figs. and described hereinafter.

The following fastener devices are preferably applicable as the disposal device 500 to the disposable pull-on garment 20 of the present invention. Although the fastener devices are joined to the nonwoven outer cover 74 of the backsheet 22 in the following embodiments described, they may be directly joined to the liquid impervious film 68 of the backsheet 22 if the nonwoven outer cover 74 is not provided in the disposable pull-on garments.

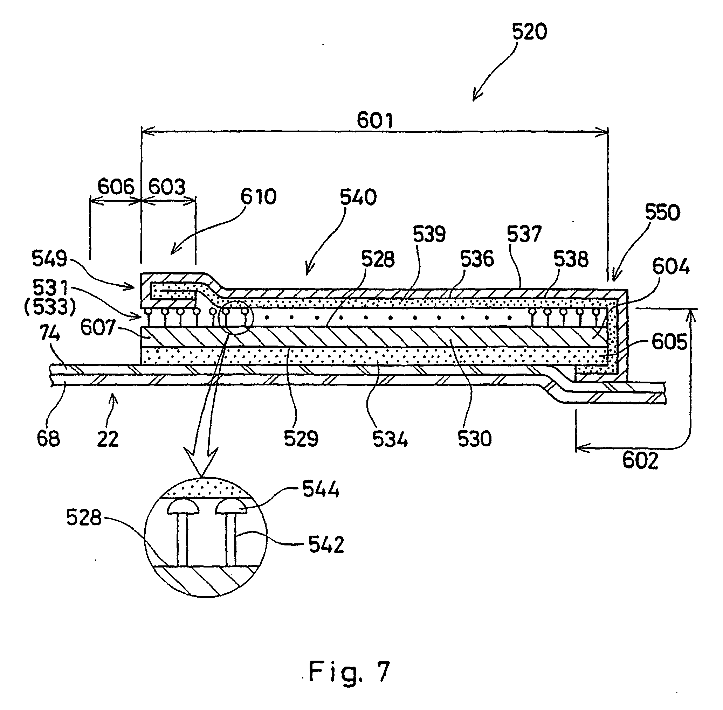

Fig. 7 is a cross-sectional view of a preferred fastener device 520. When this fastener device 520 is used as the disposal device 500 in the pull-on garment 20 shown in Fig. 6, the cross-sectional view of Fig. 7 is obtained by taking along the line 7-7 in Fig. 6. Referring to Fig. 7, the fastener device 520 includes a base 530 having a first surface 528 and a second surface 529 opposing the first surface 528; a securing means 531 provided on the first surface 528 of the base 530; and an adhesive 534 provided on the second surface 529 of the base 530. The backsheet 22 includes the liquid impervious film 68 and the nonwoven outer cover 74.

In the embodiment shown in Fig. 7, the fastener device 520 is joined to the nonwoven outer cover 74 of the backsheet 22. The base 530 is joined to a part of the nonwoven outer cover 74 through the adhesive 534. Preferably, the adhesive 534 is a layer of adhesive for securing at least a part of, more preferably, almost all of the second surface 529 of the base 530 to the nonwoven outer cover 74. The adhesive 534 is any of those adhesives which provide an adequate bond with the nonwoven outer cover 74. Preferably, the adhesive 534 is a pressure-sensitive adhesive well-known to those of ordinary skill in the adhesive art.

In a preferred embodiment, the securing means 531 is a hook material which includes first engaging elements 533 which are mechanically engageable with a loop fastening material. The first engaging elements 533 extend from the first surface 528 of the base 530. The first engaging elements 533 may take any configuration as are known by the skilled person in the art so long as they are able to engage a loop fastening material. Each of the engaging elements 533 preferably includes a stem 542 supported at one end on the first surface 528 of the base 530 and a head 544 positioned at the other end of the stem 542. The head 544 can take any shape as are known in the art so long as it is adapted to engage a loop fastening material. In a preferred embodiment, the head 544 has an enlarged shape as shown in Fig. 7.

The first engaging elements 533 may be manufactured from a wide range of materials. Suitable materials include nylon, polyester, polyethylene, polypropylene, or any combination of these materials. Preferred hook materials which include the engaging elements 533 are disclosed in, for example, U.S. Patent 4,894,060 entitled "Disposable Diaper With Improved Hook Fastener Portion" issued to Nestegard on January 16, 1990.

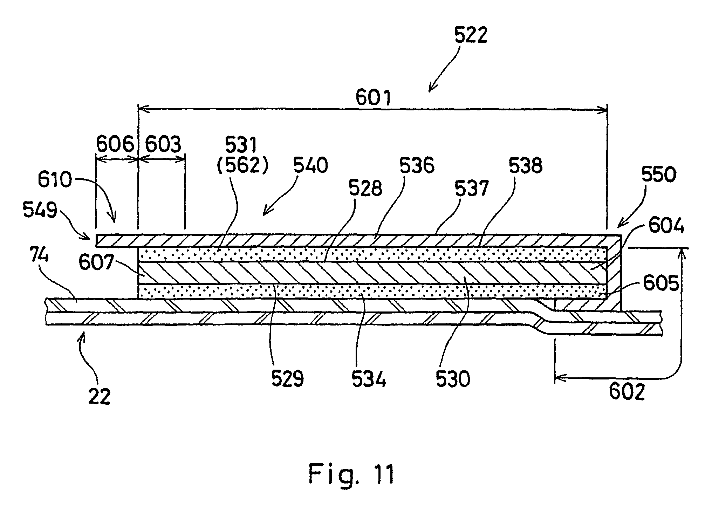

The securing means 531 may be formed by any other material than the hook material, which can be used for securing at least a part of, more preferably, all area of the first surface 528 of the base 530 to an object material to be secured, for example, an adhesive. In a preferred embodiment, an adhesive 562 (not shown in Fig. 7 but Fig. 11) is provided on the first surface 528 of the base 530 as the securing means 531.

The base 530 may be manufactured from a wide range of materials. Suitable materials include nylon, polyester, polyethylene, polypropylene, or any combination of these materials. Preferably, the base 530 is in the form of a thin plastic film of one of these materials. A preferred combined material for the base 530 and the engaging elements 533 is available from The Minnesota Mining and Manufacturing Company, St. Paul, Minn., under Code No. CS-200.

The fastener device 520 further includes a cover member 540 which has a protective region 601 and an anchor region 602. Herein, "protective region" refers to a part of the cover member 540 that is positioned on the securing means 531 so as not to expose the securing means 531. Preferably, at least a part of the cover member 540 is joined to the securing means 531 so as not to expose the securing means 531 prior to an intended use of the securing means 531. The anchor region 602 is folded to enclose one edge portion 550 of the base 530 and the securing means 531, and is joined to a part of the second surface 529 of the base 530 through the adhesive 534. This structure provide a hingedly removable feature of the cover member 540 around the one edge portion 550 of the base 530 and the securing means 531. The securing means 531 can be exposed by detaching and removing the protective region 601 of the cover member 540 from the securing means 531 when the user wants to use the securing means 531. The anchor region 602 can be joined to any part of the second surface 529 of the base 530 through the adhesive 534. In a preferred embodiment, the fastener device 520 has the anchor region 602 joined to one end portion 604 of the base 530 through the corresponding end portion 605 of the adhesive 534 as shown in Fig. 7. Preferably, the ratio of the protective region 601 to the anchor region 602 is from about 3:1 to about 10:1, more preferably from about 4:1 to about 5:1.

In a preferred embodiment, the cover member 540 includes a protection member 536 having a first surface 537 and a second surface 538 opposing the first surface 537. The protection member 536 may be manufactured from a wide range of materials. Suitable materials include nylon, polyester, polyethylene, polypropylene, or any combination of these materials. Preferred material for the protection member 536 is in the form of a thin film or a nonwoven of these materials. A suitable combined material for the protection member 536 and the adhesive 539 is available from The Minnesota Mining and Manufacturing Company, St. Paul, Minn., under Code No. KJ-5173L.

More preferably, the cover member 540 further includes an adhesive 539 provided on the second surface 538 of the protection member 536. In a preferred embodiment, the adhesive 539 is a layer of adhesive for securing at least a part of, more preferably, all of the second surface 538 of the protection member 536 to the securing means 531 before the use of the fastener device 520. The adhesive 539 can be any of those adhesives which provide an adequate bond with the securing means 531. Preferably, after the securing member 531 is exposed by removing the cover member 540, the adhesive 539 can work as a secondary or supplemental securing means which can adhesively secure to the object material that is to be secured by the securing means 531. In a preferred embodiment, the adhesive 539 is a pressure-sensitive adhesive well-known to those of ordinary skill in the adhesive art.

In the embodiment shown in Fig. 7, since the fastener device 520 can be formed by uniform component members or materials, i.e., the base 530, the securing means 531, the adhesive 534 and the cover member 540, the manufacturing process for making the fastener device 520 can be simplified. Thus, the cost for the fastener device 520 can be reduced.

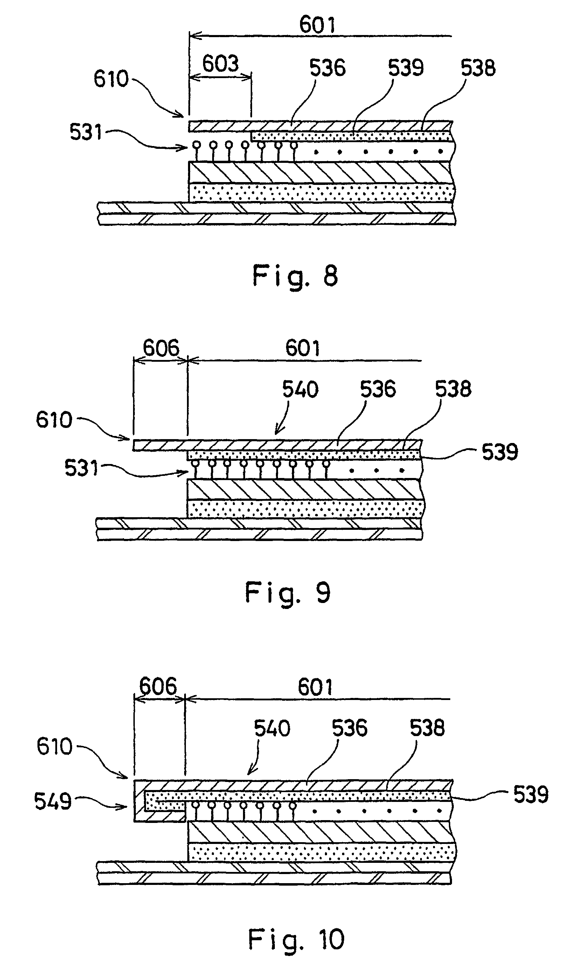

In a preferred embodiment, the cover member 540 further has a tab region 603 which provides a tab portion 610 the user of the fastener device 520 can readily grasp with the fingers when the user wants to remove the cover member 540 to expose the securing means 531. Herein, "tab region" is defined as a part of the protection member 536 which is not joined to the securing means 531. The tab region 603 (thus, the tab portion 610) is formed by not providing an adhesive on a specific area of the second surface 538 of the protection member 536, or by making inactive the adhesive provided on a specific area of the second surface 538 of the protection member 536. In one embodiment, a separate piece of plastic film or nonwoven is attached to cover the adhesive in such specific area to make the adhesive inactive. More preferably, the cover member 540 is folded back onto the second surface 538 of the cover member 540 to make the adhesive inactive as shown in Figs. 7 and 10.

In a preferred embodiment, the tab region 603 is provided within the protective region 601 as shown in Fig. 7. More specifically, while the tab region 603 is positioned within the protective region 601, it is not joined to the securing means 531 thereby forming the tab portion 610. In the embodiment shown in Fig. 7, the tab region 603 is formed by folding the free end portion 549 of the cover member 540 back onto the second surface 538 of the cover member 540. Alternatively, the tab region 603 (thus the tab portion 610) can be formed within the protective region 601 by simply eliminating the adhesive 539 from the second surface 538 of the protection member 536 as shown in Fig. 8.

Yet alternatively, the tab region 603 can be provided at an outside region 606 of the protective region 601. Referring to Fig. 9, the cover member 540 has the outside region 606 which is not joined to the securing means 531 thereby forming the tab portion 610, while the protective region 601 is joined to the securing means 531. As shown in Fig. 9, although the cover member 540 does not have the adhesive 539 on the second surface 538 of the protection member 536 in the outside region 606, the cover member 540 can have the adhesive 539 there for the purpose of a material simplification to be used (not shown in Figs.). In a preferred embodiment, a separate piece of plastic film or nonwoven is attached to the adhesive 539 provided in the outside region 606 to cover the adhesive 539 (not shown in Figs.). In an yet alternative embodiment, the free end portion 549 of the cover member 540 is folded back onto the second surface 538 of the cover member 540 in the outside region 606 thereby forming the tab portion 610 as shown in Fig. 10.

Fig. 11 is a cross-sectional view of another preferred fastener device 522. Referring to Fig. 11, the fastener device 522 includes a base 530 having a first surface 528 and a second surface 529 opposing the first surface 528; a securing means 531 provided on the first surface 528 of the base 530; and an adhesive 534 provided on the second surface 529 of the bases 530.

The base 530 is joined to the nonwoven outer cover 74 of the backsheet 22 through the adhesive 534. Preferably, the adhesive 534 is a layer of adhesive for securing at least a part of, more preferably, almost all of the second surface 529 of the base 530 to the nonwoven outer cover 74. The adhesive 534 is any of those adhesives which provide an adequate bond with the nonwoven outer cover 74. Preferably, the adhesive 534 is a pressure-sensitive adhesive well-known to those of ordinary skill in the adhesive art.

In the embodiment shown in Fig. 11, the securing means 531 is an adhesive 562 provided on the first surface 528 of the base 530. Preferably, the adhesive 562 is a layer of adhesive for adhesively securing at least a part of, more preferably, all area of the second surface 528 of the base 530 to an object material, for example, a plastic film, a nonwoven, and the like (not shown in Figs.). In a preferred embodiment, the adhesive 562 is a pressure-sensitive adhesive well-known to those of ordinary skill in the adhesive art.

The base 530 may be manufactured from a wide range of materials. Suitable materials include nylon, polyester, polyethylene, polypropylene, or any combination of these materials. Preferably, the base 530 is in the form of a thin film of one of these materials. A preferred combined material for the base 530, the adhesives 531 and 534 is available from The Minnesota Mining and Manufacturing Company, St. Paul, Minn., under Code No. LS-200.

The fastener device 520 further includes a cover member 540 which has a protective region 601 and an anchor region 602. The cover member 540 has the protective region 601 positioned on the securing means 531. The protective region 601 is joined to the securing means 531 so as not to expose the securing means 531 prior to an intended use of the securing means 531 (i.e., the adhesive 562). The anchor region 602 is folded to enclose one edge portion 550 of the base 530 and the securing means 531, and is joined to a part of the second surface 529 of the base 530 through the adhesive 534. This structure provides a hingedly removable feature of the cover member 540 around the one edge portion 550 of the base 530 and the securing means 531. The securing means 531 can be exposed by detaching and removing the protective region 601 of the cover member 540 from the securing means 531 when the user wants to use the securing means 531. The anchor region 602 can be joined to any part of the second surface 529 of the base 530 through the adhesive 534. In a preferred embodiment, the fastener device 522 has the anchor region 602 joined to one end portion 604 of the base 530 through the corresponding end portion 605 of the adhesive 534 as shown in Fig. 11. Preferably, the ratio of the protective region 601 to the anchor region 602 is from about 3:1 to about 10:1, more preferably from about 4:1 to about 5:1.

In a preferred embodiment, the cover member 540 includes a protection member 536 having a first surface 537 and a second surface 538 opposing the first surface 537. The protection member 536 may be manufactured from a wide range of materials. Suitable materials include nylon, polyester, polyethylene, polypropylene, or any combination of these materials. Preferred material for the protection member 536 is in the form of a thin film or a nonwoven of these materials. A suitable combined material for the protection member 536 and the adhesive 539 is available from The Minnesota Mining and Manufacturing Company, St. Paul, Minn., under Code No. KJ-5173L.

In a preferred embodiment, the cover member 540 further has a tab region 606 which provides a tab portion 610 the user of the fastener device 522 can readily grasp with the fingers when the user wants to remove the cover member 540 to expose the securing means 531. The tab portion 610 is formed by not providing an adhesive on a specific area of the first surface 528 of the base 530, or by making inactive the adhesive provided on a specific area of the first surface 528 of the base 530. In one embodiment, a separate piece of plastic film or nonwoven is attached to cover the adhesive in such specific area to make the adhesive inactive.

In the embodiment shown in Fig. 11, the tab region 603 is provided at an outside region 606 of the protective region 601. More specifically, the cover member 540 has the outside region 606 which is not joined to the securing means 531 thereby forming the tab portion 610, while the protective region 601 is joined to the securing means 531.

In an alternative preferred embodiment, the tab region 603 is provided within the protective region 601 (not shown in Figs.). More specifically, while the tab region 603 is positioned within the protective region 601, the adhesive 562 is eliminated in the tab region 603. Thus, the protection member 536 is not joined to the securing means 531 in the tab region 603 thereby forming the tab portion 610.

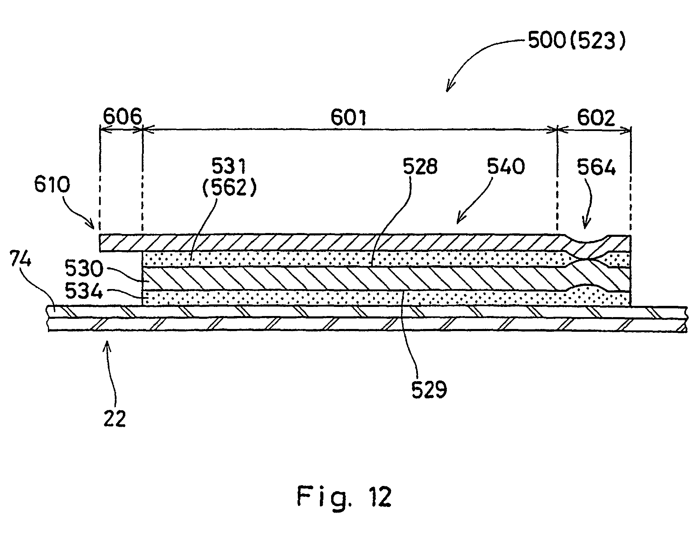

Fig. 12 is a cross-sectional view of yet another preferred fastener device 523. This fastener device 523 has a similar structure to the fastener device 522 shown in Fig. 11 except the manner for forming a hingedly removable cover member structure. Referring to Fig. 12, the fastener device 523 includes a base 530 having a first surface 528 and a second surface 529 opposing the first surface 528; a securing means 531 provided on the first surface 528 of the base 530; and an adhesive 534 provided on the second surface 529 of the bases 530. The securing means 531 is an adhesive 562 provided on the first surface 528 of the base 530. The base 530 is joined to the nonwoven outer cover 74 of the backsheet 22 through the adhesive 534.

The fastener device 523 further includes a cover member 540 which has a protective region 601, an anchor region 602 and an outside tab region 606. The protective region 601 is positioned on and joined to the securing means 531 by the adhesive 562 so as not to expose the securing means 531 prior to an intended use of the securing means 531. A tab portion 610 is formed by the cover member 540 in the outside tab region 606.

In a preferred embodiment, the cover member 540 includes a bonding portion 564 in the anchor region 602 as shown in Fig. 12. The cover member 540 is hingedly joined to the base 530. More specifically, the cover member 540 is bonded to the base 530 at the bonding portion 564. Any methods known by the skilled person can be taken to bond the cover member 540 to the base 530. In a preferred embodiment, the cover member 540 has the bonding portion 564 bonded to a part of the base 530 by an application of a pressure at a melting point or a softening temperature of either material of the base 530 or the cover member 540 between the base 530 and the cover member 540.

Although all of the above described fastener devices have the hingedly removable cover member structure, this structure may be eliminated in other embodiments. For example, the cover member 540 may be a separate member which is just attached to the first securing means 531 by the adhesive 531 but is not fixed to any part of the disposal device 500 (and the disposable pull-on garment 20). Thus, in such embodiment the cover member 540 is detached and removed from the adhesive 531 (i.e., the disposal device 500) and thrown away when the disposal device 500 is used.

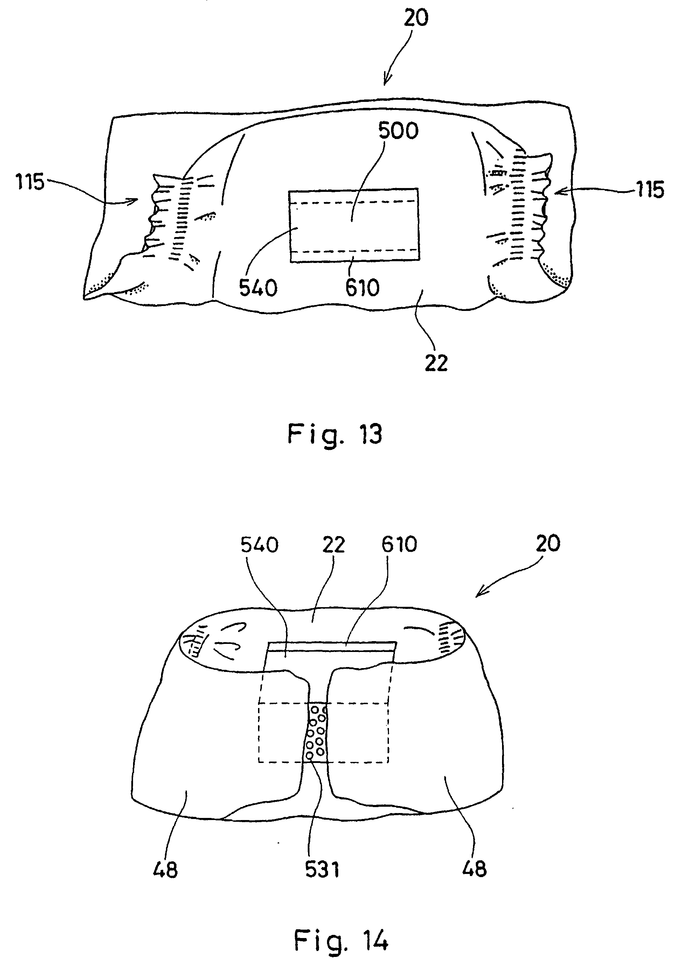

After the pull-on garment 20 has been soiled, the soiled garment 20 is torn open along the seams 32 by gripping the tear open tab 31 and the ear panel 46 or 48 to remove the soiled garment 20 from the wearer. The garment 20 is then folded or rolled up so that the disposal device 500 can come to the position for a convenient disposal as shown in Fig. 13, while containing the contents within the soiled garment 20. The cover member 540 of the disposal device 500 is then higedly opened to exposed the securing means 531 (not shown in Fig. 13 but Fig. 14) by gripping the tab portion 610 of the disposal device 500. Each part of the back ear panels 48 are then joined to the backsheet 22 through the securing means 531 to form the configuration that provides a convenient disposal as shown in Fig. 14. The configuration shown in Fig. 14 is a preferred example of a convenient disposal since all portions of the side edges 115 (not shown in Fig. 14 but Figs. 6 and 13) of the pull-on garment 20 can be wrapped within the ear panels 48 thus preventing the contents in the soiled garment 20 from leaking out over the side edges 115 of the garment 20.

In the following, preferred mechanical fastener devices which has a folded structure are described in detail. These fastener devices also can be used as the disposal device 500.

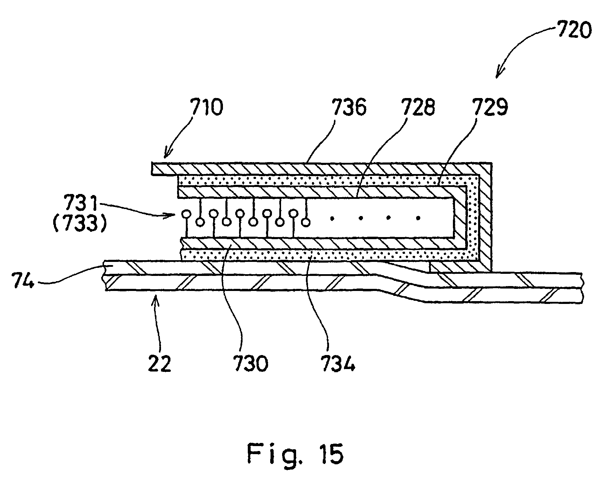

Fig. 15 is a cross-sectional view of yet another preferred fastener device 720. When this fastener device 720 is used as the disposal device 500 in the pull-on garment 20 shown in Fig. 6, the cross-sectional view of Fig. 15 is obtained by taking along the line 15-15 in Fig. 6. Referring to Fig. 15, the mechanical fastener device 720 includes a base 730 which has a first surface 728 and a second surface 729 opposing the first surface 728; a securing means 731 which includes a plurality of engaging elements 733 extending from the first surface 728 of the base 730; an adhesive 734 provided on the second surface 729 of the base 730 and an outer member 736 provided on a part of the adhesive 734. The base 730 is folded such that the engaging elements 733 mechanically engage by themselves to maintain the mechanical fastener device 720 in a folded state until manually unfolded to expose the engaging elements 733. Preferably, the mechanical fastener device 720 further has a tab portion 710 which is formed by an extended portion of the outer member 736 as shown in Fig. 15. In the embodiment shown in Fig. 15, the mechanical fastener device 720 is joined to the nonwoven outer cover 74 of the backsheet 22 through the adhesive 734.

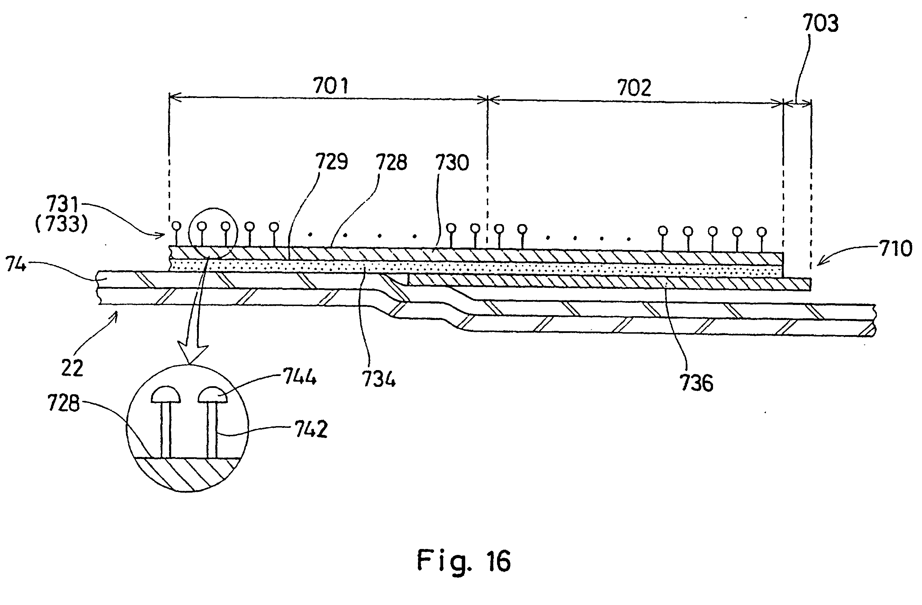

Fig. 16 is a cross-sectional view of the unfolded structure of the mechanical fastener device 720 shown in Fig. 15. Referring to Fig. 16, the base 730 has the first surface 728 and the second surface 729 opposing the first surface 728. The securing means 731 is provided on the first surface 728 of the base 730. The adhesive 734 is provided on the second surface 729 of the base 730. The securing means 731 includes the plurality of engaging elements 733 which are mechanically engageable with a loop fastening material.

The base 730 has a first region 701 and a second region 702. Each of the first and second regions 701 and 702 has the engaging elements 733 extended from the first surface 728 of the base 730. The base 730 can be folded along a predetermined folding line (not shown in Figs.) such that the first and second regions 701 and 702 face one another as shown in Fig. 15. The engaging elements 733 in the first region 701 mechanically engage with the engaging elements 733 in the second region 701 to maintain the mechanical fastener device 720 in a folded state until manually unfolded to expose the engaging elements 733 of the first and second regions 701 and 702.

In a preferred embodiment, the first and second regions 701 and 702 are adjacent to each other as shown in Fig. 16. Alternatively, the first and second regions 701 and 702 can be apart each other by inserting an additional region which does not contribute the engagement of the engaging elements 733 between the first and second regions 701 and 702.

The engaging elements 733 may take any configuration as are known by the skilled person in the art so long as they are able to engage each other and a loop fastening material. Each of the engaging elements 733 preferably includes a stem 742 supported at one end on the first surface 728 of the base 730 and a head 744 positioned at the other end of the stem 742. The head 744 can take any shape as are known in the art. In a preferred embodiment, the head 744 has an enlarged shape as shown in Fig. 16.

The engaging elements 733 may be manufactured from a wide range of materials. Suitable materials include nylon, polyester, polyethylene, polypropylene, or any combination of these materials. Preferred materials which include the engaging elements 733 are disclosed in, for example, U.S. Patent 4,894,060 entitled "Disposable Diaper With Improved Hook Fastener Portion" issued to Nestegard on January 16, 1990.

The base 730 may be manufactured from a wide range of materials. Suitable materials include nylon, polyester, polyethylene, polypropylene, or any combination of these materials. Preferably, the base 730 is in the form of a thin plastic film of one of these materials. A preferred combined material for the base 730 and the engaging elements 733 is available from The Minnesota Mining and Manufacturing Company, St. Paul, Minn., under Code No. CS-200.

The base 730 is joined to the nonwoven outer cover 74 of the backsheet 22 through the adhesive 734. The adhesive 734 is any of those adhesives which provide an adequate bond with the nonwoven outer cover 74. Preferably, the adhesive 734 is a pressure-sensitive adhesive well-known to those of ordinary skill in the adhesive art.

The outer member 736 may be manufactured from a wide range of materials. Suitable materials include nylon, polyester, polyethylene, polypropylene, or any combination of these materials. Preferred material for the outer member 736 is in the form of a thin film or a nonwoven of these materials. A suitable combined material for the outer member 736 is available from The Minnesota Mining and Manufacturing Company, St. Paul, Minn., under Code No. KJ-5173L.

In a preferred embodiment, the outer member 736 is extended from the second region 702 to the third region 703 thereby forming a tab portion 710 the user of the mechanical fastener device 720 can readily grasp with the fingers when the user wants to expose the securing means 731 for use.

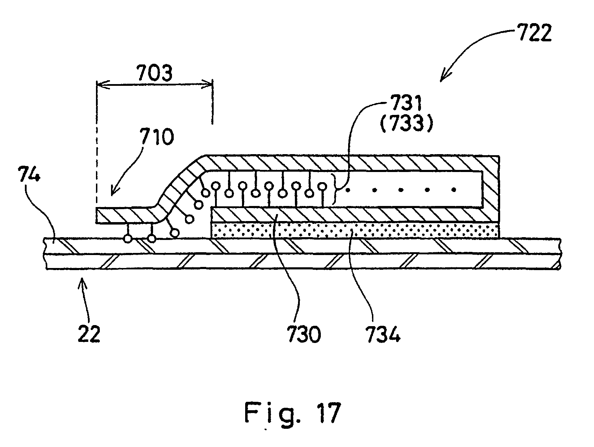

Fig. 17 is a cross-sectional view of still another preferred mechanical fastener device 722. In this embodiment, the mechanical fastener device 722 is joined to the nonwoven outer cover 74 of the backsheet 22 through the adhesive 734. The mechanical fastener device 722 includes a base 730; a securing means 731 which includes a plurality of engaging elements 733; and an adhesive 734. The engaging elements 733 extend from the base 730. The base 730 is folded such that the engaging elements 733 in the first and second regions 701 and 702 (not shown in Fig. 17) mechanically engage by themselves to maintain the mechanical fastener device 722 in a folded state until manually unfolded to expose the engaging elements 733.

In the embodiment shown in Fig. 17, the base 730 further has the third region 703 which extends from the second region 702 (not shown in Fig. 702). In the third region 703, at least a part of the engaging elements 733 provided therein engages with the loop fastening material of the disposable pull-on garment 20 until the mechanical fastener device 722 is manually unfolded to expose the engaging elements 733 in the first and second regions 701 and 702 for use. In a preferred embodiment, the third region 703 has a portion which does not have engaging elements 733 thereby forming the tab portion 710 as shown in Fig. 17.

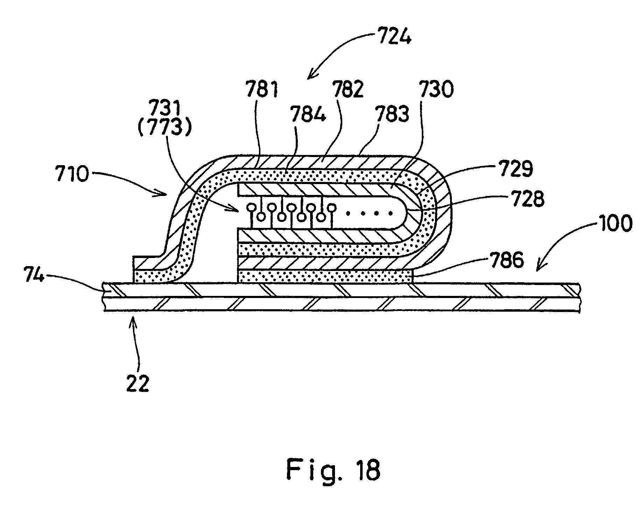

Fig. 18 is a cross-sectional view of yet another preferred mechanical fastener device 724. The mechanical fastener device 724 is joined to the nonwoven outer cover 74 of the backsheet 22 through the adhesive 786. The mechanical fastener device 724 includes a base 730 having a first surface 728 and a second surface 729 opposing the first surface 728, and a securing means 731 having engaging elements 733 extended from the first surface 728 of the base 730. The mechanical fastener device 724 further includes an outer cover member 782 having a first surface 781 and a second surface 783 opposing the first surface 781, and an adhesive 784 provided on the first surface 781 of the outer cover member 782. The second surface 729 of the base 530 is joined to the outer cover member 782 by the adhesive 784.

The outer cover member 782 may be manufactured from a wide range of materials. Suitable materials include nylon, polyester, polyethylene, polypropylene, or any combination of these materials. Preferred material for the outer cover means 782 is a strip of a thin film or a nonwoven. In a preferred embodiment, the outer cover means 782 is a plastic film strip. In another preferred embodiment, the outer cover means 582 is a nonwoven strip.

Preferably, the adhesive 784 is a layer of adhesive for joining at least a part of, more preferably, all of the second surface 729 of the base 730 to the outer cover member 782. Thus, the adhesive 784 is any of those adhesives which provide an adequate bond with the base 730 and the outer cover member 782, and preferably is any of pressure-sensitive adhesive well-known to those of ordinary skill in the adhesive art.

In the embodiment shown in Fig. 18, the outer cover member 782 is extended over the base 730 and adhesively joined to the nonwoven outer cover 74 of the backsheet 22 through the adhesive 784 until the mechanical fastener device 724 is manually unfolded to expose the engaging elements 733 for use. The extended portion of the outer cover member 782 forms the tab portion 710 in this embodiment.

## Claims

1. A disposable pull-on garment (20;120) having a front region (26) a back region (28) and a crotch region (30) between the front region (26) and the back region (28), comprising:
a chassis (41) provided in the front, back and crotch regions, the chassis (41) including a liquid pervious topsheet (24), a liquid impervious backsheet (22) associated with the topsheet (24), and an absorbent core (25) disposed between the topsheet (24) and the backsheet (22),
at least one pair of ear panels (45,46,48) extending laterally outward from the chassis (41) in the front (26) or back region (28), the ear panels (45,46,48) being joined to the chassis (41) to form two leg openings (34) and a waist opening (36), and **characterized in that**
a disposal device (500) joined to the backsheet (22) in the crotch region (30), the disposal device (500) including a first securing means (531) which is capable of securing the disposable pull-on garment (120) in a convenient disposal configuration after the garment has been soiled, wherein the convenient disposal configuration is formed by securing a part of the ear panels (45,46,48) to the backsheet (22) through the first securing means (531),
wherein the disposal device (500) has a means (540) for protecting the first securing means (531) from an exposure prior to an intended use of the first securing means (531).

2. The disposable pull-on garment (20;120) of Claim 1, wherein the means (540) for protecting the first securing means (531) is a cover member provided on the first securing means (531).

3. The disposable pull-on garment (20;120) of Claim 1, whecein the disposable pull-on garment (20;120) has side edges (115), and wherein the side edges (115) can be wrapped within the ear panels (45,46,48) in the convenient disposal configuration.

4. The disposable pull-on garment (20;120) of Claim 1, wherein the first securing means (531) is a plurality of engaging elements which can mechanically engage with a part of the ear panels (46,45,48).

5. The disposable pull-on garment (20;120) of Claim 1, wherein the first securing means (531) is a first adhesive which can secure a part of the ear panels (45,46,48) thereto.

6. The disposable pull-on garment (20;120) of Claim 2, wherein the cover member (540) is hingedly joined to the disposal device (500).

7. The disposable pull-on garment (20;120) of Claim 6, wherein the disposal device (500) further includes a second adhesive provided between the cover member (540) and the first securing means (531), and wherein the second adhesive acts as a second securing means which can secure a part of the ear panels (45,46,48) thereto when the cover means (548) is hingedly removed to expose the first securing means (531).

8. The disposable pull-on garment (20;120) of Claim 4, wherein the ear panel (45,46,48) includes a nonwoven material, and the plurality of engaging elements are mechanically engageable with the nonwoven material.

9. The disposable pull-on garment (20;120) Claim 8, wherein the ear panel (45,46,48) is formed from a "zero strain" stretch laminate.

10. The disposable pull-on garment (20;120) according to Claim 1, wherein the ear panels (45,46,48) are extensible at least in the lateral direction.

## Patentansprüche

1. Wegwerfbare Anziehwäsche (20; 120) mit einer vorderen Region (26), einer hinteren Region (28) und einer Schrittregion (30) zwischen der vorderen Region (26) und der hinteren Region (28) mit:
einem Grundkörper (41), der in der vorderen, der hinteren und der Schrittregion vorgesehen ist, wobei der Grundkörper (41) eine flüssigkeitsdurchlässige Oberschicht (24), eine mit der Oberschicht (24) verbundene flüssigkeitsundurchlässige Unterschicht (22) und einen absorbierenden Kern (25), der zwischen der Oberschicht (24) und der Unterschicht (22) angeordnet ist, umfaßt,
wenigstens einem Paar Flügelfelder (45, 46, 48), die sich seitlich von dem Grundkörper (41) in der vorderen (26) und der hinteren Region (28) nach außen erstrekken, wobei die Flügelfelder (45, 46, 48) mit dem Grundkörper (41) so verbunden sind, daß sie zwei Beinöffnungen (34) und eine Taillenöffnung (36) bilden, und **dadurch gekennzeichnet, daß**
eine Wegwerfeinrichtung (500), die mit der Unterschicht (22) in der Schrittregion (30) verbunden ist, wobei die Wegwerfeinrichtung (500) ein erstes Befestigungsmittel (531) umfaßt, welches in der Lage ist, die wegwerfbare Anziehwäsche (120) in einer bequemen Wegwerfkonfiguration festlegt, nachdem die Wäsche beschmutzt worden ist, wobei die bequeme Wegwerfkonfiguration geformt wird, indem ein Teil der Flügelfelder (45, 46, 48) durch das erste Befestigungsmittel (531) an der Unterschicht (22) festgelegt wird;
wobei die Wegwerfeinrichtung (500) im Mittel (540) zum Schützen des ersten Befestigungsmittels (531) vor einer Freilegung vor der gedachten Verwendung des ersten Befestigungsmittels (531) hat.

2. Wegwerfbare Anziehwäsche (20; 120) nach Anspruch 1, in welcher das Mittel (540) zum Schützen des ersten Befestigungsmittels (531) ein Abdeckelement ist, welches auf dem ersten Befestigungsmittel (531) vorgesehen ist.

3. Wegwerfbare Anziehwäsche (20; 120) nach Anspruch 1, in welcher die wegwerfbare Anziehwäsche (20; 120) Seitenränder (115) hat, und wobei die Seitenränder (115) in die Flügelfelder (45, 46, 48) in die bequeme Wegwerfkonfiguration gewickelt werden können.

4. Wegwerfbare Anziehwäsche (20; 120) nach Anspruch 1, in welcher das erste Befestigungsmittel (531) eine Mehrzahl von eingreifenden Elementen ist, welche mechanisch in einen Teil der Flügelfelder (46, 45, 48) eingreifen können.

5. Wegwerfbare Anziehwäsche (20; 120) nach Anspruch 1, in welcher das erste Befestigungsmittel (531) ein erstes Haftmittel ist, welches einen Teil der Flügelfelder (45, 46, 48) daran festlegen kann.

6. Wegwerfbare Anziehwäsche (20; 120) nach Anspruch 2, in welcher das Abdekkelement (540) mit der Wegwerfeinrichtung (500) gelenkig verbunden ist.

7. Wegwerfbare Anziehwäsche (20; 120) nach Anspruch 6, in welcher die Wegwerfeinrichtung (500) ferner ein zweites Haftmittel aufweist, das zwischen dem Abdeckelement (540) und dem ersten Befestigungsmittel (531) vorgesehen ist, und wobei das zweite Haftmittel als ein zweites Befestigungsmittel wirkt, welches einen Teil der Flügelfelder (45, 46, 48) daran festlegen kann, wenn das Abdeckmittel (548) gelenkig so bewegt wird, daß das erste Befestigungsmittel (531) freigelegt ist.

8. Wegwerfbare Anziehwäsche (20; 120) nach Anspruch 4, in welcher das Flügelfeld (45, 46, 48) ein Vliesmaterial umfaßt und die Mehrzahl der eingreifenden Elemente in das Vliesmaterial mechanisch eingreitbar sind.

9. Wegwerfbare Anziehwäsche (20; 120) nach Anspruch 8, in welcher das Flügelfeld (45, 46, 48) aus einem "Nullspannung"-Stretchlaminat gebildet ist.

10. Wegwerfbare Anziehwäsche (20; 120) nach Anspruch 1, in welcher die Flügelfelder (45, 46, 48) wenigstens in der Querrichtung dehnbar sind.

## Revendications

1. Vêtement à enfiler jetable (20 ; 120) comportant une région avant (26), une région arrière (28) et une région d'entrejambe (30) entre la région avant (26) et la région arrière (28), comprenant :
un châssis (41) prévu dans les régions avant, arrière et d'entrejambe, le châssis (41) comprenant une feuille de dessus (24) perméable aux liquides, une feuille de fond (22) imperméable aux liquides associée à la feuille de dessus (24), et une âme absorbante (25) placée entre la feuille de dessus (24) et la feuille de fond (22),
au moins une paire de panneaux à oreilles (45, 46, 48) s'étendant latéralement vers l'extérieur à partir du châssis (41) dans la région avant (26) ou arrière (28), les panneaux à oreilles (45, 46, 48) étant réunis au châssis (41) afin de former deux ouvertures de jambe (34) et une ouverture de ceinture (36) ; **caractérisé en ce que**
un dispositif de mise au rebut (500) est réuni à la feuille de fond (22) dans la région d'entrejambe (30), le dispositif de mise au rebut (500) comprenant un premier moyen d'assujettissement (531) qui est capable d'assujettir le vêtement à enfiler jetable (120) dans une configuration pratique de mise au rebut après que le vêtement a été souillé, dans lequel la configuration pratique de mise au rebut est formée en assujettissant une partie des panneaux à oreilles (45, 46, 48) à la feuille de fond (22) par l'intermédiaire du premier moyen d'assujettissement (531) ;
dans lequel le dispositif de mise au rebut (500) comporte un moyen (540) pour protéger le premier moyen d'assujettissement (531) d'une exposition avant une utilisation prévue du premier moyen d'assujettissement (531).

2. Vêtement à enfiler jetable (20 ; 120) selon la revendication 1, dans lequel le moyen (540) pour protéger le premier moyen d'assujettissement (531) est un élément de recouvrement prévu sur le premier moyen d'assujettissement (531).

3. Vêtement à enfiler jetable (20 ; 120) selon la revendication 1, dans lequel le vêtement à enfiler jetable (20 ;120) présente des bords latéraux (115) et dans lequel les bords latéraux (115) peuvent être enveloppés à l'intérieur des panneaux à oreilles (45, 46, 48) dans la configuration pratique de mise au rebut.

4. Vêtement à enfiler jetable (20 ; 120) selon la revendication 1, dans lequel le premier moyen d'assujettissement (531) est une pluralité d'éléments de prise qui peuvent venir en prise mécaniquement avec une partie des panneaux à oreilles (45, 46, 48).

5. Vêtement à enfiler jetable (20 ; 120) selon la revendication 1, dans lequel le premier moyen d'assujettissement (531) est un premier adhésif qui peut assujettir une partie des panneaux à oreilles (45, 46, 48) à celui-ci.

6. Vêtement à enfiler jetable (20 ; 120) selon la revendication 2, dans lequel l'élément de recouvrement (540) est réuni de manière articulée au dispositif de mise au rebut (500).

7. Vêtement à enfiler jetable (20 ; 120) selon la revendication 6, dans lequel le dispositif de mise au rebut (500) comprend, en outre, un deuxième adhésif prévu entre l'élément de recouvrement (540) et le premier moyen d'assujettissement (531), et dans lequel le deuxième adhésif agit comme un deuxième moyen d'assujettissement qui peut assujettir une partie des panneaux à oreilles (45, 46, 48) à celui-ci lorsque le moyen de recouvrement (548) est retiré de manière articulée afin d'exposer le premier moyen d'assujettissement (531).

8. Vêtement à enfiler jetable (20 ; 120) selon la revendication 4, dans lequel le panneau à oreille (45, 46, 48) comprend un matériau non tissé, et la pluralité d'éléments de prise peut venir en prise mécaniquement avec le matériau non tissé.

9. Vêtement à enfiler jetable (20 ; 120) selon la revendication 8, dans lequel le panneau à oreille (45, 46, 48) est formé à partir d'un stratifié étiré de « déformation zéro ».

10. Vêtement à enfiler jetable (20 ; 120) selon la revendication 1, dans lequel les panneaux à oreilles (45, 46, 48) sont extensibles au moins dans la direction latérale.
